(19) **Europäisches Patentamt / European Patent Office / Office européen des brevets**

(11) **EP 1 916 994 B1**

(12) **EUROPEAN PATENT SPECIFICATION**

(45) Date of publication and mention
of the grant of the patent:
**11.12.2013 Bulletin 2013/50**

(51) Int Cl.:
*A61K 9/00* (2006.01)   *A61K 9/20* (2006.01)
*A61K 31/542* (2006.01)

(21) Application number: **05753592.4**

(86) International application number:
**PCT/DK2005/000435**

(22) Date of filing: **28.06.2005**

(87) International publication number:
**WO 2006/000228 (05.01.2006 Gazette 2006/01)**

(54) **MANUFACTURING OF QUICK RELEASE PHARMACEUTICAL COMPOSITION OF WATER INSOLUBLE DRUGS AND PHARMACEUTICAL COMPOSITIONS OBTAINED BY THE PROCESS OF THE INVENTION**

HERSTELLUNG EINER PHARMAZEUTISCHEN ZUSAMMENSETZUNG MIT SCHNELLER FREISETZUNG AUS WASSERUNLÖSLICHEN ARZNEIMITTELN UND MIT DEM ERFINDUNGSGEMÄSSEN VERFAHREN ERHALTENE PHARMAZEUTISCHE ZUSAMMENSETZUNGEN

PREPARATION DE COMPOSITIONS PHARMACEUTIQUES DE MEDICAMENTS INSOLUBLES A L'EAU A DIFFUSION RAPIDE ET COMPOSITIONS PHARMACEUTIQUES AINSI PREPAREES

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **29.06.2004 DK 200401021**

(43) Date of publication of application:
**07.05.2008 Bulletin 2008/19**

(73) Proprietor: **Takeda Pharma A/S
4000 Roskilde (DK)**

(72) Inventor: **BERTELSEN, Poul
DK-4000 Roskilde (DK)**

(74) Representative: **Plougmann & Vingtoft A/S
Rued Langgaards Vej 8
2300 Copenhagen S (DK)**

(56) References cited:
EP-A- 1 352 660   WO-A-00/15195
WO-A-01/41536   WO-A-99/09988
WO-A-99/12524   WO-A1-2004/047824
US-A- 4 689 218

• MURA P ET AL: "Solid-state characterization and dissolution properties of Naproxen-Arginine-Hydroxypropyl-beta-cyclo dextrin ternary system" EUROPEAN JOURNAL OF PHARMACEUTICS AND BIOPHARMACEUTICS, ELSEVIER SCIENCE PUBLISHERS B.V., AMSTERDAM, NL, vol. 59, no. 1, January 2005 (2005-01), pages 99-106, XP004658790 ISSN: 0939-6411

Remarks:
The file contains technical information submitted after the application was filed and not included in this specification

EP 1 916 994 B1

**Description**

**FIELD OF INVENTION**

The present invention relates to the field of pharmaceutical formulation science, in particular with respect to methods of improving the solubility and dissolution of water-insoluble drugs. The present invention is especially focused on compositions comprising a drug substance belonging to the class of drug substances normally denoted NSAID's (non-steroidal anti-inflammatory drug). In particular NSAID's with low solubility in water and gastric fluid, and with stability problems are of interest. An example of such an NSAID is lornoxicam. However, other drug substances having a low solubility in acidic medium and/or a $pK_a$ below about 5.5, may as well be suitable for being formulated in a composition according to the invention. The present invention provides oral dosage forms with a significantly improved stability.

**BACKGROUND**

Fast absorption of drugs into the circulating blood is generally required in managing pain relieves. Therefore, for oral dosage forms it is of utmost importance to have the drug dissolved, completely or partly, already when present in the gastric fluid. Thus, in the event where the drug is not absorbed from the gastric mucosa, it may be ready for being absorbed already when entering the upper intestinal tract, such as duodenum. Duodenum itself has a limited amount of liquid, thus resulting in slow dissolution of the drug in duodenum, although the weak acid may be more soluble in the intestinal fluid.

Several approaches have been reported for the manufacturing and the formulation of oral dosage forms of drugs that are substantially water-insoluble and are weak acids so as to achieve quick in vitro dissolution in gastric fluid.

Some references have taken the approach of improving the solubility of an NSAID by forming an inclusion complex with cyclodextrin. WO 9641646 relates to a parenteral formulation of lornoxicam formulated as an inclusion complex with cyclodextrin. The medicament is either present as a powder for reconstitution or as a solution. All examples imply a solvating step and the reference is silent of preparing the formulation without the use of water.

Likewise, WO 9532737 relates to an inclusion complex of an NSAID, such as lornoxicam and a cyclodextrin. The manufacturing process includes a wetting step so the reference does necessarily imply the use of water in the formulation process.

WO9909988 discloses pharmaceutical compositions of meloxicam prepared by modification of the solid state of the drug, said compositions being stated as having improved dissolution behaviour over a broad range of pH. Example IV/12 discloses dry co-milling wherein the ratio between drug and alkaline substance is about 20:1. It appears that addition of cyclodextrin results in improvement of solubility and bioavailability. WO9909988 does not provide any information that the molar ratio between the NSAID and alkaline substance is important in order to achieve an improved dissolution of stable oral formulations of NSAID.

Other references have taken other approaches for improving the solubility of an NSAID. For example, it is steadily reported that such water insoluble drugs need to be formulated together with an alkaline substance. It is further reported that the manufacturing process should include contacting the drug-containing and alkaline containing powder with an aqueous medium to form a particulate composition.

EP 1109534 B1 discloses a formulation comprising a therapeutically and/or prophylactically active substance, which has a solubility of at the most about 0.1 % w/v in 0.1 N hydrochloric acid at room temperature, the composition being based on a powder that comprises the active substance and an alkaline substance and which are being contacted with an aqueous medium to form a particulate composition. Such compositions have a dissolution rate in 0.1 N hydrochloric acid such that at least 50% of the drug is present on dissolved form within the first 20 minutes of dissolution testing. Importantly, it is stated that the fast dissolution rate disclosed in this patent is not achieved if the active substance and the alkaline substance are processed under conditions where an aqueous contact between the two components does not take place (i.e. under anhydrous conditions).

WO 9912524 relates to a modified release multiple-unit formulation where the active substance is an NSAID. The formulation is characterised in having two fractions of multiple-units where one fraction is fast-releasing and the other is slow releasing. The fast releasing fraction corresponds to the fast release formulation of reference EP 1109534 (WO 15195) cited above.

JP 3240729 and EP 792147 also relate to formulations wherein the active compound is granulated together with an alkaline substance using an aqueous solution.

However, until now it is the understanding that the use of aqueous solutions during the manufacturing process impose separate problems for drugs that are unstable in the presence of water and an alkaline substance. On the other hand, the prior art clearly teaches that wet-granulation is needed to provide a fast in vitro dissolution. Therefore, there is a need for providing formulations, still possessing fast dissolution in hydrochloric acid, while at the same time exhibiting a good stability.

## SUMMARY OF INVENTION

Now provided is a pharmaceutical composition, intended for oral administration, and manufactured by a process utilising a minimum of liquid, preferably without utilising liquid at all and utilising intensive mixing in the form of co-milling so as to provide close physical contact between the active drug substance and a dissolution helper (alkaline substance).

Though the oral dosage form was manufactured without using any liquid, the resulting batches had a water content on the same level as batches produced by means of wet-granulation.

Surprisingly, the batches produced according to the invention had a significantly improved stability irrespective of the water content.

Remarkably, the batches resulting from the co-milling process still processed a fast *in vitro* dissolution: The active drug substance has a fast *in vitro* dissolution rate at conditions simulating the gastric fluid, so that that at least 50% of the active drug substance is dissolved within the first 20 minutes of in vitro dissolution testing.

Accordingly, in a first aspect the present invention is directed to a process for manufacturing an oral dosage form comprising an active drug substance having a fast dissolution in gastric fluid; said process comprising the steps of:

a) providing the active drug substance;
b) providing one or more alkaline substance(s); and
c) mixing said active drug substance and said alkaline substance(s) and optionally one or more excipients by co-milling without adding a liquid, and optionally
d) admixing one or more pharmaceutically acceptable excipients, and optionally
e) compressing said mixture of c) or d) into a tablet;

wherein the active drug substance has a solubility at room temperature of less than 0.1% w/v in 0.1 N hydrochloric acid or has a pKa value less than 5.5; and wherein the molar ratio between said active drug substance and said alkaline substance(s) ranges between 1:100 and 1:1.

In a second aspect the present invention concerns a pharmaceutical composition obtainable by the process of the invention.

In a further aspect the present invention is directed to a stable pharmaceutical composition for oral administration comprising:

- an NSAID or a pharmaceutically acceptable salt or prodrug thereof;
- one or more alkaline substances selected from a salt containing an anion selected from $CO_3^{2-}$, $HPO_4^{2-}$, $PO_4^{3-}$ and a cation selected from $Na^+$ and $K^+$;
- and a binder in the form of a hydrophilic polymer;

wherein said pharmaceutical composition has been manufactured by a process utilising intensive mixing in the form of co-milling and without utilising liquid at all wherein the molar ratio between said NSAID or pharmaceutically acceptable salt or prodrug thereof and said alkaline substance(s) or a derivative thereof ranges between 1:100 and 1:1.

In a still further aspect the present invention concerns a stable pharmaceutical composition for oral administration comprising:

- an NSAID or a pharmaceutically acceptable salt or prodrug thereof; and
- one or more amino acids or a derivative thereof;

wherein said pharmaceutical composition has been manufactured by a process comprising mixing said NSAID or pharmaceutically acceptable salt or prodrug thereof and said one or more amino acids or a derivative thereof and optionally one or more excipients by co-milling without adding a liquid, wherein the molar ratio between said NSAID or pharmaceutically acceptable salt or prodrug thereof and said amino acid(s) or a derivative thereof ranges between 1:100 and 1:1.

Thus, present invention is directed not only to methods for manufacturing of pharmaceutical compositions, but also to stable pharmaceutical compositions.

## DETAILED DESCRIPTION OF THE INVENTION

It has surprisingly been found that pharmaceutical compositions comprising water insoluble drugs can be manufactured and formulated in a manner ensuring fast dissolution of the active drug substance in the gastric fluid, while also providing conditions for improving the stability with respect to the active drug substance. In the present invention the drug substance is not exposed, or at least only to a minimum extent, to any liquid or to any aqueous solution during the manufacturing process. The composition resulting from

a process excluding water was until now believed to result in compositions with a lower water content than seen after wet-granulation. A low water content was expected to provide compositions potentially having longer shelf life than conventionally formulated and processed drugs. The water content in compositions manufactured according to the invention proved, however, to have a water content on the same level as compositions produced by means of wet-granulation which granulate was subsequently dried. Very surprisingly the present co-milling process provides a significantly improved stability despite of the water content. At the same instance the provided compositions display a fast *in vitro* dissolution, though the prior teaching emphasize that the active substance and the alkaline excipient should be contacted with water for providing a fast *in vitro* dissolution. Thus, for active substances sensitive to water, the process is advantageous.

Advantageously, the described co-milling process is economically profitable as the process requires fewer production steps. Especially, the laborious wetting step and the subsequent expensive drying step of the prior manufacturing method are avoided. Furthermore, special requirements regarded dehumidification of the air at the production facilities may be avoided.

Very surprisingly, as demonstrated in example 10 that when batches produced by means of co-milling were compared with batches produced by means of wet-granulation and where all batches had a water content on the same level irrespective of the manufacturing process, there was a significant improvement in stability of the batches manufactured by co-milling. The mechanism of the improvement in stability is not understood in detail but without being limited to a specific theory it is contemplated that the impact of the granulation liquid in case of wet-granulation alters the crystal structure of the active drug substance. This alteration of crystal structure does not take place in the compositions produced by co-milling. By the present inventor it is therefore suggested, without being limited to this theory, that when contacting a drug substance, as demonstrated with lornoxicam, with a granulation liquid the drug substance, in this case lornoxicam partly dissolves for subsequently to solidify into a less stable form. This less stable form is likely to be an amorf form of the drug substance, e.g. of the active drug substance. From the promising stability results on lornoxicam it is concluded that this process will also be advantageous for other drug substances, such as other NSAID including thiazinecarboxamides.

The improved stability was demonstrated in a stability study, as described in example 10, where co-milled compositions were compared to a batch produced by means of wet-granulation. In this study the degradation product of lornoxicam, HN-10004 was chosen as the stability indicating parameter. In this instance the co-milling was performed by means of a ball mill with horizontally moving spheres. It is, however, firmly believed that the type of co-milling will not influence the stability as the decisive factor for the stability is whether or not the active drug substance is contacted with a granulation liquid. Other co-milling procedures as described herein are therefore also suitable.

At the starting point of the test period the water-content of the tablets were determined. The water-content of all compositions was on the same level, with a tendency of the batch produced by wet-granulation to be lower in water-content than the two other compositions. Until now it has been well-known that the stability of lornoxicam in combination with an alkaline substance is closely related to a low water content in the tablet.

In the stability study the stability was tested at several test points over a period of 6 months as appears from example 10. As the stability indicating parameter was in example 10 chosen the decomposition product HN-10004. At all test points the amount of HN-10004 in the batch nos. 17110431 and 17110432 (co-milled) was lower than in batch no. 10225671 (wet-granulated).

At all the test points the stability of the two co-milled compositions are superior to a significant extent over the wet-granulated batch, despite the fact that the wet-granulated batch has a lower water-content.

At the starting point, the amount of HN-10004 is also lower in the co-milled product showing that decomposition takes place even at the time of production in the wet-granulated batches.

Very surprisingly the co-milling process thus leads to a product with a significantly improved stability irrespective of the higher water content.

The co-milling process was demonstrated with lornoxicam as a model substance, and it is most likely that this process will be suitable for other active drug substances. Such drug substances are typically weak acid where the alkaline substance will function as a dissolution aid, and the co-milling process is particularly suitable for active drug substances with stability problems.

In suitable embodiments of the invention, the oral dosage form further comprises a binder, which unlike a number of conventional manufacturing processes, has not been presented to aqueous solution during the manufacturing process or pre-treated with aqueous solution before use. Therefore, the binder is not present in swelled form, as may be determined under a microscope, e.g. a scanning electron microscope (SEM) with sufficient magnifying effect.

It is to be understood, that the manufacturing process and oral dosage forms of the invention may be characterised in terms of

- type of therapeutically active substance
- type of alkaline substance

- mixing process and the resulting mixture of the active drug substance and alkaline substance
- quick dissolution of the active drug substance in acidic solution
- stability of the therapeutically active ingredient.;

These aspects will be discussed in the following.

In some embodiments the manufacturing process and the oral dosage form of the invention may further be characterised in terms of the process of compressing tablets. E.g. for a 10mm round standard concave tablet a force of minimum app. 4 kN is applied.

*Results from the experiments*

Different equipment for co-milling can be implied in the process. Below is presented an overview of type of co-milling equipment, parameters and examples enclosed herein.

| Type of co-milling equipment | Specific type of co-milling equipment | Force applied by the equipment/parameters | Time of impact | Ex. |
|---|---|---|---|---|
| Roller compaction | Minipactor® from Gerteis Maschinen + Processengineering AG. | High force Compaction force: 8-12 kN/cm Rpm: 2 Sieve size: 1.0-1.5 mm Gab size: 2.5 mm | Short time such as less than 1 min. | 6 |
| Ball mill, vertically moving spheres | Struers ball mill | Low force 250 - 400 rpm sieve: 700$\mu$m mesh. | Long time: ½-24 hours | 11 |
| Ball mill with horizontally moving spheres | Fritsch Pulverisette type 06.002.00 | Medium force | time 5 to 30 min. | 2 3 4 10 |
| Mechano fusion | AMS-LAB mechano fusion unit from Hosokawa Alpine. | Medium force Time: 3-30 min Rotor speed: 1300 - 1500 Temp: 20-45 °C | Medium time 5 to 30 min. | 5 |
| Milling alkaline, admixture | - | - | | 7 8 |
| Simple mixture | - | - | | 9 |

Examples 2, 3, 4 and 10 relate to co-milling provided by ball milling with horizontally moving spheres. The alkaline substance used in example 2 is trisodium phosphate. Two types of binder is used; HPC and VA 64 both combinations providing a fast dissolution of the resulting tablets. The co-milling is in this instance provided by a medium force and medium impact time. In example 3 the alkaline substance is sodium carbonate and the milling process is again ball milling with horizontally moving spheres. Similarly to example 2, two types of binder is used; HPC and VA 64 both combinations providing a fast dissolution of the resulting tablets. Finally in example 4 arginine is used as the alkaline substance providing tablets with a fast release that is on the same level as that obtained with trisodium phosphate. The stability of tablets provided by the procedure described in examples 2 and 3 were tested in a stability study, example 10. The tablets displayed excellent stability compared to tablets provided by known manufacturing methods.

Another principle of co-milling also providing a medium force and a medium impact time was investigated, Mechano fusion milling. In example 5 tablets are provided after co-milling with a AMS-LAB mechano fusion unit from Hosokawa Alpine. The tablets either contained trisodium phosphate, sodium carbonate, arginine or lysine as the alkaline substance. All compositions had a fast dissolution, the batch with lysine showed outstandingly fast dissolution results. Two compositions are based on trisodium phosphate with different mean particle sizes of the alkaline substance; 203$\mu$m, 40$\mu$m respectively. Though both the batch with the large as well as the batch with the small particle size gave acceptable results, dissolution was significantly faster in the batch with the smaller particle size.

To demonstrate that a low force combined with a long impact time can provide the co-milling effect, tablets were produced with lysine or trisodium phosphate as the alkaline substance in example 5. Furthermore the batches produced with a molar ratio lornoxicam:alkaline substance of 1:20 was compared with batches produced with a molar ratio of 1:40. All batches displayed fast dissolution and the molar ratio had no impact on the dissolution rate.

Finally, a high force combined with a short impact time was provided by roller compaction. In example 6 tablets were provided by means of Minipactor® from Gerteis Maschinen + Processengineering AG using the alkaline substances trisodium phosphate or lysine. Batches were produced, based on both a small and a large mean particle size of the alkaline substance. All batches showed fast dissolution and the particle size did not seem to have major impact with this co-milling method.

Alternatively the close contact provided by co-milling can be established by milling an alkaline substance in the equipment used for co-milling or other suitable equipment followed by a simple admixture or a blending and subsequent tabletting, where the tabletting provides a high force in a short impact time.

In example 7 lysine as the alkaline substance was milled in Hosokawa Alpine spiral jet mill to a mean particle size of 5μm followed by compression of the mixture of ingredients into tablets. Two batches were produced that both showed a fast dissolution.

From the examples it is concluded that either a co-milling of the active drug substance together with the alkaline substance or a milling procedure of the alkaline substance followed by compression into tablets or alternatively a wet-granulation, as is described in the prior art, is needed to provide tablets with a fast dissolution. To demonstrate this statement, tablets were produced according to the prior teaching (e.g. JP 3240729 or EP 1109534), namely the wet-granulation process but **without** the wetting step. This experiment is described in more detail in example 9 and as is seen in the example, the resulting tablets have an unacceptable slow dissolution.

Conclusively, the examples demonstrate that a fast dissolution is provided by co-milling of a therapeutically active drug substance having a solubility at room temperature of less than 0.1% w/v in 0.1N hydrochloric acid or which has a pKa of less than 5.5 together with an alkaline substance, optionally followed by admixture of other excipients and optionally followed by tabletting of the mixture of ingredients.

Alternatively, the alkaline substance can by milling according to the methods described herein for subsequently to be mixed with the active drug substance, optionally followed by admixture of other excipients for subsequently to be compressed into tablets.

Finally the fast dissolution can be provided by co-milling of the active drug substance together with an alkaline substance and optionally together with other excipients followed by optional admixture of even further other excipients and subsequently followed by compressing the mixture of ingredients into tablets.

*Therapeutically active ingredient*

In principle any active ingredient characterised by having a poor solubility in acidic solution, may be processed by the above-mentioned manufacturing process in order to improve the dissolution in acidic solution and thus ensuring fast absorption of the drug in the upper gastrointestinal tract upon orally administering the resulting composition.

According to the present invention, the active ingredient is a therapeutically active compound having a solubility at room temperature in 0.1 N hydrochloric acid of less than 0.1% w/v. Alternatively defined, the therapeutically active compound has a pKa value of less than 5.5 in that such compounds are also known to dissolve poorly in the gastric fluid. Furthermore, the active ingredient may be defined as belonging to the group of NSAID's that are characterised by being weak acids. Examples on NSAID's are lornoxicam and naproxone.

A majority of the active drug substances mentioned are weak acids, i.e. substances which have a $pK_a$ value below about 5.5 such as, e.g., in a range of from about 3.0 to about 5.5 or in a range of from about 4.0 to about 5.0. In this connection it can be mentioned that the $pK_a$ value for lornoxicam is about 4.7, for naproxen about 4.2, for indometacin about 4.5, for ibuprofen about 5.2 and for acetylsalicylic acid about 3.5. Moreover, active drug substances like those mentioned above generally have a poor solubility in media having a pH below the $pK_a$ value. As an example the solubility of lornoxicam at a pH of 0.1 N HCl is less than about 1 mg/100 ml at room temperature. Active drug substances like acetylsalicylic acid, indometacin and naproxen are regarded as substances, which are practically insoluble in water and in 0.1 N HCl at room temperature.

The term "active drug substance" is in the present description and claims used synonymously with "therapeutically active substance", "therapeutically active ingredient" and "therapeutically active compound".

Likewise, the term "pharmaceutical composition" is in the present description and claims used synonymously with "pharmaceutical formulation", "formulation" and "dosage form".

Relevant examples of active drug substances suitable for use in compositions according to the invention are in general weak acidic substances such as, e.g., paracetamol and/or NSAID substances like

- aminoarylcarboxylic acid derivatives like e. g. enfenamic acid, flufenamic acid, isonixin, meclofenamic acid, mefenamic acid, morniflumate, niflumic acid, and tolfenamic acid,
- arylacetic acid derivatives like e.g. aceclofenac, acemetacin, amfenac, bromfenac, cimmetacin, diclofenac, etodolac, fentlazac, glucametacin, indomethacin, lonazolac, metiavinic acid, oxametacine, pirazolac, proglumetacin, sulindac, tiaramide, tolmetin, and zomepirac,
- arylcarboxylic acids like e.g. ketorolac and tinoridine,
- arylpropionic acid derivatives like e. g. alminoprofen, bermoprofen, carprofen, dexibuprofen, fenbufen, fenoprofen, flunoxaprofen, flurbiprofen, ibuprofen, ibuproxam, ketoprofen, loxoprofen, naproxen, oxaprozin, pranoprofen, protizinic acid, and tiaprofenic acid,
- pyrazoles like e.g. epirizole,
- pyrazolones like e.g. benzpiperylon, mofebutazone, oxyphenbutazone, phenylbutazone, and ramifenazone,
- salicylic acid derivatives like e.g. acetaminosalol, acetylsalicylic acid, benorylate, eterisalate, fendosal, imidazole salicylate, lysine acetylsalicylate, morpholine salicylate, parsalmide, salamidacetic acid and salsalate,
- thiazinecarboxamides like a.o. ampiroxicam, droxicam, lornoxicam, meloxicam, piroxicam, and tenoxicam,
- others like bucillamine, bucolome, bumadizon, diferenpiramide, ditazol, emorfazone, nabumetone, nimesulide, proquazone, acrivastine and piroxicam (e.g. in the form of a betacyclodextrin complex), wherein the NSAID may be in the form of a pharmaceutically acceptable salt or a prodrug.

From a market point of view especially the following NSAID's are interesting: lornoxicam, diclofenac, nimesulide, ibuprofen, piroxicam, piroxicam (betacyclodextrin), naproxen, ketoprofen, tenoxicam, meloxicam, tolfenamic acid, bromazepam, aceclofenac, indometacin, nabumetone, acemetacin, morniflumate, meloxicam, flurbiprofen, tiaprofenic acid, proglumetacin, mefenamic, acid, fenbufen, etodolac, tolfenamic acid, sulindac, phenylbutazone, fenoprofen, tolmetin, acetylsalicylic acid, dexibuprofen and pharmaceutically acceptable salts, complexes and/or prodrugs and mixtures thereof.

In particular the following NSAID's are interesting: piroxicam, meloxicam, ibuprofen, tolfenamic acid and bromazepam.

Other relevant active drug substances are COX-2 (COX is an abbreviation for cyclooxygenase) inhibitors like e.g. celecosib and flosulide.

At present, the most preferred drug substance is lornoxicam and pharmaceutically acceptable salts, complexes and/or prodrugs thereof, such as esters thereof. Lornoxicam may be present in a composition according to the invention as the sole drug substance or in combination with other drug substances such as opioids or triptan's.

Relevant examples of opioid substances are morphine, hydromorphone, codeine, oxycodone, hydrocodone, methadone, levorphanol, fentanyl, buprenorphine, butorphanol tartrate, dezocine, nalbuphine hydrochloride and meperidine.

Relevant examples of triptan substances are metoclopramide, sumatriptan, rizatriptan, naratriptan, colmitriptan, eletriptan, almotriptan, zolmitriptan and frovatriptan.

In those cases where a quick release composition of the present invention includes an NSAID substance as the therapeutically active ingredient, the amount of the active drug substance corresponds to from 1 to about 1600 mg by weight. Alternatively, the dosage form may contain molar equivalent amounts of pharmaceutically acceptable salts thereof. The dosage form contains an appropriate amount to provide a substantially equivalent therapeutic effect.

In preferred embodiments of the invention, the active ingredient is lornoxicam. This drug is a weak acid (pKa less than 5.5) and has solubility in 0.1 N HCl less than 0.1% w/v and is prone to degradation in the presence of water. The tendency of degradation in presence of water is dependent of the presence of excipients, such as alkaline substances, and are in particular dependent on the manufacturing process as was demonstrated in the present specification.

The dissolution rate of an active drug substance may further be affected by the particle size of the drug substance. Accordingly, in interesting embodiments of the invention, the active drug substance may be further defined in terms of its particle size distribution. The particle size distribution can be determined by laser diffraction (e.g. using a Malvern Mastersizer 2000). The particle size distribution is calculated according to the Frauenhofer respective Mie theories. The sample is first dispersed in tenside solution as pre-treatment method. Then an aliquot of the pre-dispersion is transferred to a dispersion bath where further particle dispersion occurs while stirring and treating with ultrasound. This suspension is circulated through the measuring cell. During measurement the stirring remain active whereas the ultrasound is switched off.

Typically, the particle size distribution as determined by the above-mentioned laser diffraction of the active drug substance, is such that at least 90% by volume has a particle size below 100 $\mu$m, preferably below 75 $\mu$m, more preferably below 50 $\mu$m, such as about 40 $\mu$m. In some embodiments the particle size distribution is such that at least 95% by volume has a particle size below 32$\mu$m, such as below 20$\mu$m or most preferably below 10$\mu$m. In other embodiments the particle size distribution is such that at least 80% by volume has a particle size below 10$\mu$m.

However, as may be understood, the use of particle sizes in the lower range may not be practically acceptable.

Thus, in still more interesting embodiments of the invention, the active drug substance has a particle size distribution, as determined by laser diffraction, wherein at least 90% by volume of the active drug substance has a particle size above 0.1 $\mu$m.

In some embodiments the mean particle size, D(v;0.5) ($\mu$m), is used. Typically, the mean particle size relates to an excipient, such as the mean particle size of an alkaline substance. By the mean particle size is understood a particle distribution determined by laser diffraction as described above, wherein the distribution of the particles are such that 50% of the particles are above and 50% of the particles are below the mean particle size, and wherein the distribution is determined by volume.

*Alkaline substance*

As stated, the oral dosage form of the invention should further comprise an alkaline substance. It is considered important that the alkaline substance is in physical contact with the active drug substance, such as lornoxicam. It is thought that the alkaline substance enables a microenvironment around the active drug substance so as to aid the dissolution of the active drug substance in acidic solutions when the composition is being exposed to acidic solution or water.

The molar ratio between the active drug substance and the alkaline substance ranges between 1:100 and 1:1, preferably, the said molar ratio is 1:80, 1:60, 1:40 or 1:30, most preferably 1:20. In still other embodiments the molar ratio of the active drug substance and the alkaline substance is 1:10. The ratio of 1:10 is especially interesting in the embodiment wherein the alkaline substance is an amino acid or a derivative thereof, e.g. lysine, histidine or arginine or a derivative thereof.

As used herein, the term an "alkaline substance" is meant to include substances that provide an alkaline pH in the range of 8-14, preferably 8-13, when being dissolved in water at room temperature in an amount of about 10mg/ml.

Accordingly, the term "alkaline substance" includes the corresponding base of an organic or an inorganic acid, such as provided in the form of a pharmaceutically acceptable salt of an organic or inorganic acid and mixtures thereof, organic amines and some amino acids or derivatives thereof. Typically, the organic or inorganic acid from where the corresponding base derives has a pKa in the range of 4 -14.

Relevant alkaline substances are listed in table 1.

**Table 1: List of alkaline substances**

| Substance | Examples | Structure | pKa[*] |
|---|---|---|---|
| Salts of carbonic acid (carbonates and hydrogencarbonates) and phosphoric acid (phosphates and mono hydrogenphosphates). Soluble salts with pKa-values from 4 to 11 | Di-sodium carbonate = Sodium carbonate | $Na_2CO_3$ | 10.3 |
| | Sodium hydrogencarbonate = Sodium bicarbonate | $NaHCO_3$ | 6.4 |
| | Tri-sodium phosphate | $Na_3PO_4$ | 12.4 |
| | Di-sodium hydrogen-phosphate | $Na_2HPO_4$ | 7.2 |
| Salts of organic acids with pKa-values from 4 to 11 | Sodium acetate | $CH_3COONa$ | 4.8 |
| | Sodium citrate | $C_6H_5O_7Na_3$ | 6.4 |
| | Sodium maleate | $C_4H_2O_4Na_2$ | 6.2 |
| | Sodium fumerate | $C_4H_2O_4Na_2$ (trans) | 4.4 |
| Organic amines | Hydroxylamine | $NH_2OH$ | 6 |
| | Diethyl amine | $(CH_3CH_2)_2NH$ | 11 |
| | Triethyl amine | $(CH_3CH_2)_3N$ | 10.8 |
| | Hydrazine | $NH_2NH_2$ | 8 |
| | Codeine | $C_{18}H_{21}NO_3$ | 8.2 (pH in saturated solution $\approx$ 9.8) |

(continued)

| Substance | Examples | Structure | pKa* |
|---|---|---|---|
| Amino acids with $pKa_3$-values from 8 to 14 | Lysine | $C_6H_{14}O_2N_2$ | $pKa_1$: 2.2<br>$pKa_2$: 8.9<br>$pKa_3$: 10.3 |
| | Arginine | $C_6H_{14}N_4O_2$ | $pKa_1$: 2.2<br>$pKa_2$: 9.1<br>$pKa_3$: 13.2<br>$pH\approx11.4$<br>(100 g/L $H_2O$) |
| | Histidine | $C_6H_9O_2N_3$ | $pKa_1$: 1.8<br>$pKa_2$: 6.0<br>$pKa_3$: 9.0<br>$pH\approx7.7$<br>(10 g/L $H_2O$) |

* The pKa-values in this table are approximate values and refer to the pKa of the acid.

In one embodiment of the present invention, the alkaline substance is a salt of an organic or inorganic acid or a mixture thereof, the organic or inorganic acid has a pKa in the range of 4-14, preferably in the range of 6 to 13.5, even more preferable in the range of 7-13, most preferably in the range of 8-13, such as 8.5-13, such as 9-13, such as 9-12,5.

In some embodiments of the present invention the alkaline substance is a salt of an inorganic acid selected from carbonic acid or phosphoric acid, such as hydrogencarbonic acid, dihydrogenic phosphoric acid and hydrogenic phosphoric acid.

That is to say that the salt has as the anion, an anion selected from carbonate, phosphate, and hydrogenphosphate group and as the kation, an earth metal selected from sodium, potassium, calcium, magnesium and the like, e.g. a salt containing an anion selected from $CO_3^{2-}$, $HPO_4^{2-}$, $PO_4^{3-}$ and a kation selected from $Na^+$, $K^+$, $Ca^{2+}$, $Mg^{2+}$.

Typically, the salt of an inorganic acid is selected from di-sodium carbonate, di-sodium hydrogenphosphate and tri-sodium phosphate. Typically, hydrogencarbonate salts are not so feasible because of the effervescent effect unless this is an object.

In yet some embodiments of the present invention the alkaline substance is a salt of an organic acid, the organic acid being selected from citric acid, maleric acid or acetic acid. That is to say wherein the salt has an anion selected from acetate, hydrogencitrate, citrate, hydrogenmaleate or maleate. Typically, the salt of an organic acid is sodium acetate, trisodium citrate, disodiumhydrogencitrate or disodium maleate, and preferably trisodium citrate.

In still another embodiment of the present invention, the alkaline substance is an organic amine selected from hydroxylamine, diethyl amine, triethyl amine or hydrazine.

In still another embodiment of the present invention the alkaline substance is an amino acid such as histidine, lysine or arginine.

It is further contemplated that the alkaline substance should be soluble in water, such as at least to comply with the category of being sparingly soluble in water: 1 part of alkaline substance can be dissolved in a maximum of 100 parts of water. Preferably, the alkaline substance should be soluble in water: 1 part of alkaline substance is dissolved in a maximum of 30 parts of water.

Furthermore, it should be understood that the alkaline substance should be provided in solid form, such as in the form of a powder, granulate or the like.

*Process*

As mentioned, the invented process includes a step of mixing the active drug substance and the alkaline substance by co-milling without adding a liquid. Importantly, the said mixing step has one object, namely to ensure the close physical contact between the active drug substance and the alkaline agent so as to achieve the desired microenvironment. To ensure the close physical contact, it may be appropriate not to add further excipients and to use the active drug substance and the alkaline substance in molar ratios ranging between 1:100 and 1:1. Preferably, the said molar ratio is 1:80, 1:60, 1:40 or 1:30, most preferably 1:20.

Typically, it is not an object of the said mixing procedure to affect the particle size of the active drug substance. In some instances, it may be expected that the intensive mixing may decrease the particle size of the alkaline substance,

EP 1 916 994 B1

when being applied in particle sizes above 100 μm.

Importantly, the mixing should be undertaken under conditions excluding the addition of liquid such as aqueous liquids, water, mixtures of organic solvents and water, so as to provide conditions potentially reducing the degradation of the active drug substance. Thus, any step of conventional wet-granulation is excluded from the process.

The mixing is carried out by co-milling, which transfers energy to the mixture of active drug substance and alkaline agent so as to bring the active drug substance and the alkaline substance in close physical contact, much closer than expected with conventional mixing under formation of a particulate matter. The resulting particulate matter comprises the active drug substance and alkaline substance substantially homogeneously mixed within each other, but not molecularly dispersed within each other. Thus, it should be understood that the resulting particulate matter contains each of the constituents (alkaline substance and drug substance) as separate particles.

By the term "co-milling" is meant a highly intensive mechanical mixing of two or more substances which bring these two substances in close physical contact with each other, closer than by using a conventional mixing procedure such as tumble mixing procedure.

The term "co-milling" as used herein is also meant to include any process achieving the same particulate matter as that obtained by co-milling, for example the mixing provided under dry granulation e.g. roller compaction as discussed herein.

The co-milling process is preferably applied only to the active drug substance and the alkaline substance, but lower amounts of other ingredients may be added in the case that the fast dissolution is achieved. In the embodiments where the alkaline substance has a tendency of sticking, which was observed in relation with using some amino acids as the alkaline substance, it is advantageous to add lower amounts of other ingredients such as calcium monohydrogen phosphate, anhydrous ($CaHPO_3$), trisodium phosphate ($Na_3PO_4$), magnesium aluminium silicate, magnesium oxide, calcium carbonate ($CaCO_3$), calcium sulphate dihydrate ($CaSO_4$, $2H_2O$), sorbitol or talc. In a preferred embodiment, the co-milling is carried out on a mixture consisting essentially of the active drug substance and the alkaline substance.

In one embodiment the milling is performed only on the alkaline substance and the milling is provided by the same methods as the co-milling or other suitable equipment. The milling is typically resulting in a mean particle size of the alkaline substance in the range of 1 to 400μm, such as 1 to 300μm, such as 5 to 200μm. Following the milling of the alkaline substance this substance is admixed with the active drug substance having a particle size distribution of at least 95% by volume has a particle size below 32μm, such as below 20μm or most preferably below 10μm. In other embodiments the particle size distribution is such that at least 80% by volume has a particle size below 10μm. By admixing is intended a mixing procedure where the procedure does not imply a significant force on the mixture but only has the aim of mixing the components. The admixing is followed by compression into tablets, which compression has the effect of bringing the active dug substance and the alkaline substance into such a close contact that is otherwise provided by co-milling.

Co-milling can be achieved by using standard milling equipment, such as Hammer Mill (e.g. Fitz Mill, supplied by Fitz Patrick). The co-milling process may also be carried out using a Ball Mill (e.g. Fritz Pulverizette), which is a ball mill with horizontally moving spheres. Another principle is a ball mill having vertically moving spheres, such as a Struers ball mill also available at Hosokawa. A mechano fusion equipment (as supplied by Hosokawa) or a Micros Ring Mill. Finally, can a roller compactor provide co-milling, e.g. Minipactor® from Gerteis Maschinen + Processengineering AG.

By the term "co-milling" is thus understood a process that results in creating a close physical contact between lornoxicam and an alkaline substance. This contact can be created by use of a relatively high force combined with a relatively short period of impact as when using roller compaction. When using roller compaction the compaction force is typically in the range of 6-14 kN/cm with an impact time of less than 1 minute. Compaction of tablets also provides a relatively high force combined with a relatively short period of impact. Tabletting typically provides a force of 4kN or more for a standard concave round 10mm tablet and an impact time of less than 1 minute. Alternatively using a relatively low force combined with a longer time of impact can provide the same degree of co-milling. As an example, ball mill with vertically moving spheres provides a low force, in the latter case a considerably longer time of impact is required. The use of an intermediate amount of time of impact is also possible when a medium force is provided by mechano fusion or ball milling with horizontally moving spheres. If the alkaline substance has a small particle size, the physical contact can be created by a simple mixing and subsequent compression into tablets. Said small particle size can be obtained by milling.

Ball milling

Co-milling performed by ball milling can be divided into ball milling performed with horizontally moving spheres or with vertically moving spheres. Utilising ball milling performed with horizontally moving spheres provides a medium force intensity and thus needs a medium time of impact, such as 5 to 30 minutes. An example of an equipment suitable for performing ball milling with horizontally moving spheres is Fritz Pulverizette. Ball milling with vertically moving spheres provides a low force and therefore requires a long impact time. An example of equipment suitable for performing ball milling with vertically moving spheres is Struers ball mill.

Mechano fusion

The basic operation principle is to circulate a powder by a rotor while receiving a strong force when meeting a press head. This procedure is repeated at high speed thereby forming the powder into a particulate matter. An example of equipment is AMS-LAB mechano fusion unit from Hosokawa Alpine.

Roller Compaction

The working principle of roller compaction is to press powder between 2 counter rotating rollers to make a solid sheet which is subsequently crushed in a sieve to form a particulate matter. In this particulate matter a close mechanical contact between the powder has been obtained. An example of equipment is Minipactor® from Gerteis Maschinen + Processengineering AG.

By the term "micronised" as used herein is intended particles having a mean particle size below app. 5 μm resulting from a milling process.

By the term "rpm" is to be understood "rotations pr. minute". The term is typically used to describe the number of revolution of a moveable part of equipment such as the blade of a mixing equipment or the paddle of a dissolution equipment.

By the term "sieve size" is to be understood the diameter of the mesh of a sieve.

By the term "RH" is to be understood the "relative humidity", typically describing the amount of water vapour present in the air at a defined temperature.

In principle, the resulting mixture of the active drug substance and the alkaline substance (the co-milled mixture) of the invention can be used directly for making orally administrable dosage forms. That is to say, without the addition of further pharmaceutically acceptable excipients.

However, in some embodiments of the invention the process comprises a second step comprising admixing of one or more pharmaceutically acceptable excipients to said particulate matter using conventional mixing, such as by tumble mixing. Thus, an oral dosage form of the invention may comprise one or more further pharmaceutically acceptable excipients, such as filler (diluent), binder, disintegrant, glidant, colours and so forth. Often the dosage form will comprise a filler, or a binder, or a disintegrant, or a glidant, or a colour or a combination of one or more of the excipients.

For example, the further pharmaceutically acceptable excipient may be selected with the object of providing an oral dosage form in the form of a tablet, a pill, a capsule, a sachet or the like.

Accordingly, in one preferred embodiment of the Invention, the process further comprises the step of compressing the particulate matter under tabletting conditions so as to achieve a tablet. Compressing a powder into a particulate matter improves the flowability by tabletting, which may further improve the dissolution of the active substance.

In one embodiment the further pharmaceutically acceptable excipients is a binder, preferably a hydrophilic binder selected from cellulose derivatives, saccharine or povidone. Typically, any binder can be applied as long as the resulting tablet has a disintegration time in water at 37°C of less than 30 minutes, preferably less than 15 minutes, more preferably less than 5 minutes.

*Dissolution Properties*

In contrast to what was previously known, it was shown evident that a fast dissolution of an active drug substance can be obtained by utilising a process excluding the addition of liquid, such as aqueous liquid, to a particulate matter that comprises the active drug substance and the alkaline substance.

Therefore, in one embodiment of the invention, the resulting particulate matter or the mixture of ingredients of the active drug substance and alkaline substance, optionally in admixture with further excipients, has an *in vitro* dissolution profile, when being subjected to a dissolution test method using 1300 ml of 0.1 N HCl or 0.07N HCl equilibrated at 37 °C as the dissolution medium and USP paddle dissolution apparatus II applied with a stirring rate of 50 rpm, characterised in that at least 50% w/w of the active substance is present on dissolved form in the dissolution medium at the time point of 20 minutes after start of the dissolution testing.

In another embodiment a dissolution test method was applied using a stirring rate of 150 rpm but otherwise maintaining the same parameters as described above is applied. The in *vitro* dissolution profile of the pharmaceutical composition is characterised in that at least 50% w/w of the active substance is present on dissolved form in the dissolution medium at the time point of 20 minutes after start of the dissolution testing.

Preferably, the resulting particulate matter or the mixture of ingredients has an *in vitro* dissolution profile, characterised in that at least 55% w/w, such as at least 60% w/w, at least 65% w/w, at least 70% w/w, at least 75% w/w, or at least 80% w/w of the active substance is present on dissolved form in the dissolution medium at the time point of 20 minutes after start of the dissolution testing.

As stated, further pharmaceutically acceptable excipients may be added to the particulate matter, for instance with the object to further improve the dissolution rate.

In preferred embodiments of the invention, the particulate matter is compressed into a tablet. The present inventor provides herein evidence that the further step of compressing the particulate matter provides an even faster dissolution of lornoxicam in 0.1 N HCl.

Thus, in a further embodiment, the resulting tablet has an *in vitro* dissolution profile, when being subjected to dissolution test method using 0.1 N HCl equilibrated at 37 °C as the dissolution medium and USP paddle dissolution apparatus applied with a stirring rate of 50 rpm as the equipment, characterised in that at least 75% w/w of the active substance is present on dissolved form in the dissolution medium at the time point of 20 minutes after start of the dissolution testing.

Preferably the resulting tablet has an *in vitro* dissolution profile, characterised in that at least 80% w/w, such as at least 85% w/w, at least 90% w/w, at least 95% w/w of the active substance is present on dissolved form in the dissolution medium at the time point of 20 minutes after start of the dissolution testing.

*Stability*

The oral dosage form of the invention has a shelf life at least as good as the one processed with the addition of aqueous liquids. During the development phase it has surprisingly become evident that the stability of the co-milled compositions are significantly better than conventionally produced compositions, despite the water content being on the same level.

Therefore, a particular embodiment of the invention relates to an oral dosage form comprising lornoxicam in physical contact with an alkaline substance, wherein the oral dosage form is chemically stable with respect to the lornoxicam, such that at least 85% by weight of the lornoxicam is present in the oral dosage after at least 3 months of storage at 25 °C and 60 % RH in darkness. The storage may be performed in closed containers, blister packaging material, or in open containers. Preferably, at least 85% w/w, more preferably 90% w/w, even more preferably at least 95% w/w, most preferably at least 98% w/w of the lornoxicam is present in said composition or dosage unit after at least 6 months, preferably more than 12 months, even more preferably more than 24 months and most preferably more than 36 months of storage at the above-mentioned conditions.

Another way to define the stability aspect of the invention relates to the concentration of degradation products or impurities in the said oral dosage form of lornoxicam. The concentration of degradation products present in the oral dosage is determined after at least 3 months, such as at 3 months, of storage at 25 °C and 60 % RH in darkness. The storage may be performed In closed containers, blister packaging material, or in open containers. Preferably, the total sum of degradation products in the oral dosage form amounts to less than 15 % of the Initial amount of lornoxicam, more preferably less than 10 %, even more preferably less than 5 %, most preferably less than 2 % w/w.

This allows for a shelf life of more than 6 months, preferably more than 12 months, even more preferably for more than 24 months and most preferably more than 36 months.

There is reason to believe that when formulating a composition according to the invention using other active drug substances, such as NSAIDs, e.g. thiazinecarboxamides that these other active drug substances will also meet the above described stability requirements.

*Further Excipients*

As noted above, the oral dosage form of the invention may comprise a number of additional pharmaceutically acceptable excipients other than the alkaline substance, such as solvents, surfactants, binders, fillers, disintegrants, coatings, diluents, glidants, stabilisers, lubricants, artificial sweeteners, flavouring agents, buffering agents or colorants.

Disintegrating agents may be incorporated such as e.g. alginic acid - alginates, carboxymethylcellulose calcium, carboxymethylcellulose sodium, crospovidone, hydroxypropylcellulose, hydroxypropylmethylcellulose (HPMC), cellulose derivatives such as low-substituted hydroxypropylcellulose (e.g LH 11, LH 20, LH 21, LH 22, LH 30, LH 31, LH 32 available from Shin-Etsu Chemical Co.) and microcrystalline cellulose, polacrilin potassium or sodium, polyacrylic acid, polycarbofil, polyethylene glycol, polyvinylacetate, polyvinylpyrrolidone (e.g. Polyvidon® CL, Polyvidon® CL-M, Kollidon® CL, Polyplasdone® XL, Polyplasdone® XL-10); sodium carboxymethyl starch (e.g. Primogel® and Explotab®), sodium croscarmellose (i.e. cross-linked carboxymethylcellulose sodium salt; e.g. Ac-Di-Sol®), sodium starch glycolate, starches (e.g potato starch, maize starch, rice starch), pregelatinised starch.

Those skilled in the art will appreciate that it is desirable for compressible tablets to disintegrate within 30 minutes, more desirable within 15min, most desirable within 5 min; therefore, the disintegrant used preferably results in the disintegration of the tablet within 30 minutes, more preferable within 15 min, most preferable within 5 min.

Fillers/diluents/binders may be incorporated such as e.g. dextrins, maltodextrins (e.g. Lodex® 5 and Lodex® 10), dextrose, fructose, glucose, inositol, erythritol, isomalt, lactitol, lactose (e.g., spray-dried lactose, α-lactose, β-lactose, Tabletose®, various grades of Pharmatose®, Microtose or Fast-Floc®), maltitol, maltose, mannitol, sorbitol, sucrose, tagatose, trehalose, xylitol, low-substituted hydroxypropylcellulose (e.g LH 11, LH 20, LH 21, LH 22, LH 30, LH 31, LH 32 available from Shin-Etsu Chemical Co.), microcrystalline cellulose (e.g., various grades of Avicel®, such as Avicel® PH101, Avicel® PH102 or Avicel® PH105, Elcema® P100, Emcocel®, Vivacel®, Ming Tai® and Solka-Floc®), starches or modified starches (e.g potato starch, maize starch, rice starch, pre-gelatinised starch), polyvinylpyrrolidone, polyvinylpyrrolidone/vinyl acetate copolymer, agar (e.g. sodium alginate), calcium hydrogen phosphate, calcium phosphate (e.g. basic calcium phosphate, calcium hydrogen phosphate), calcium sulphate, carboxyalkylcellulose, dextrates, dibasic calcium phosphate, gelatine, gummi arabicum, hydroxypropyl cellulose, hydroxypropylmethylcellulose, magnesium carbonate, magnesium chloride, methylcellulose, polyethylene glycol, polyethylene oxide, polysaccharides e.g. dextran, soy polysaccharide, sodium carbonate, sodium chloride, sodium phosphate.

Glidants and lubricants may be incorporated such as stearic acid, metallic stearates, talc, waxes and glycerides with high melting temperatures, hydrogenated vegetabable oils, colloidal silica, sodium stearyl fumarate, polyethylenglycols and alkyl sulphates.

Suitable lubricants include talc, magnesium stearate, calcium stearate, stearic acid, hydrogenated vegetable oils and the like. Preferably, magnesium stearate is used.

Surfactants may be incorporated such as non-ionic (e.g., polysorbate 20, polysorbate 21, polysorbate 40, polysorbate 60, polysorbate 61, polysorbate 65, polysorbate 80, polysorbate 81, polysorbate 85, polysorbate 120, sorbitane monoisostearate, sorbitanmonolaurate, sorbitan monopalmitate, sorbitan monostearate, sorbitan monooleate, sorbitan sesquioleate, sorbitan trioleate, glyceryl monooleate and polyvlnylalkohol), anionic (e.g., docusate sodium and sodium lauryl sulphate) and cationic (e.g., benzalkonium chloride, benzethonium chloride and cetrimide) or mixtures thereof. Other appropriate pharmaceutically acceptable excipients may include colorants, flavouring agents, and buffering agents.

A film coating may also be applied on a composition according to the invention provided that the coating does not substantially retard the release of the active drug substance from the composition, but only to increase the swallowability, appearance, stability or in order to minimize any dusty problems.

Film coatings may be applied such as e.g. as hydroxypropylmethylcellulose (HPMC) (e.g. HPMC E5, HPMC E15), hydroxyethylcellulose, hydroxypropylcellulose, polydextrose and maltodextrin, Sepifilm™ and Sepifilm™ LP available from Seppic S.A., Pharmacoat® available from Shin-Etsu Chemical Co.

Film additives may be incorporated such as e.g. acetylated monoglyceride, acetyltributyl, acetyltributyl citrate, acetyltriethyl citrate, benzyl benzoate, calcium stearate, castor oil, cetanol, chlorebutanol, colloidal silica dioxide, dibutyl phthalate, dibutyl sebacate, diethyl oxalate, diethyl malate, diethyl maleate, diethyl malonate, diethyl fumarate, diethyl phthalate, diethyl sebacate, diethyl succinate, dimethylphthalate, dioctyl phthalate, glycerin, glyceroltributyrate, glyceroltriacetate, glyceryl behanate, glyceryl monostearate, hydrogenated vegetable oil, lecithin, leucine, magnesium silicate, magnesium stearate, polyethylene glycol, propylene, glycol, polysorbate, silicone, stearic acid, talc, titanium dioxide, triacetin, tributyl citrate, triethyl citrate, zinc stearate, wax.

DEFINITIONS OF SELECTED TERMS USED HEREIN

The term "shelf-life" is intended to mean the period of time, wherein the therapeutically active substances in a composition is stable at ambient conditions, e.g. 25°C and 60% RH (relative humidity), such that at least 90%, preferably 95%, more preferably 98% of the initial amount of said substances is still present in the composition within the specified shelf-life.

The terms "quick release", "fast release" or "enhanced release" in the present context refer to a modified release composition of which the release of the active drug substance and its subsequent absorption are fast. More specifically, the terms "quick release", "fast release" or "enhanced release" mean that for a composition - when subjected to a dissolution method as described above wherein at least about 50% w/w of the active substance is dissolved within the first 20 min of the test.

The term "formulated" is intended to relate to the selection of excipients, carriers, vehicles, solvents, co-solvents, preservatives, colouring agents, flavouring agents and so forth in the preparation of a medicament using said composition. The term "formulated" is furthermore intended to relate to the selection of the device for delivery of the composition or selection of containment device for administration or storing of the composition.

In the present context, the term "pharmaceutically acceptable excipient" is intended to denote any material, which is inert in the sense that it substantially does not have any therapeutic and/or prophylactic effect *per se.* A pharmaceutically acceptable excipient may be added to the active drug substance with the purpose of making it possible to obtain a pharmaceutical formulation, which has acceptable technical properties.

The terms "NSAID's" or "NSAID substances" are used herein to designate a group of drugs that belongs to non-steroid anti-inflammatory drug substances and pharmaceutically acceptable salts, prodrugs and/or complexes thereof as well as mixtures thereof.

The terms "opioids" or "opioid substances" are used herein to designate a group of substances, pharmaceutically acceptable salts, prodrugs and/or complexes thereof as well as mixtures thereof that are used in the management of moderate to severe pain because of their effectiveness, ease of titration, and favourable risk-to-benefit ratio. Opioids produce analgesia by binding to specific receptors both within and outside the CNS.

The terms "triptans" or triptane substances" are used herein to designate a group of drug substances ad pharmaceutically acceptable salts, prodrugs and/or complexes thereof as well as mixtures thereof that act as agonists for 5-hydroxytryptamine (5-HT) receptors. The triptans are often very effective in relieving migraine but do not prevent future attacks or lessen their frequency.

*Further embodiments*

In one aspect a pharmaceutical composition obtainable by the process according to the invention, where the pharmaceutical composition in one embodiment comprises:

- an NSAID; and
- an amino acid or a derivative thereof.

In another embodiment

- the active drug substance is ampiroxicam, droxicam, lornoxicam, meloxicam, piroxicam, tenoxicam, bromazepam, ibuprofen, tolfenamic acid or dexibuprofen or a pharmaceutically acceptable salt or prodrug thereof;
- the alkaline substance is histidine, lysine or arginine.

In another embodiment

- the active drug substance is lornoxicam or a pharmaceutically acceptable salt or prodrug thereof;
- the alkaline substance is histidine, lysine or arginine.

In another embodiment

- the active drug substance is lornoxicam;
- the alkaline substance is histidine, lysine or arginine.

In a further aspect
A stable pharmaceutical composition for oral administration comprises:

- an NSAID or a pharmaceutically acceptable salt or prodrug thereof;
- one or more alkaline substances selected from a salt containing an anion selected from $CO_3^{2-}$, $HPO_4^{2-}$, $PO_4^{3-}$ and a cation selected from $Na^+$ and $K^+$;
- and a binder in the form of a hydrophilic polymer;

wherein said pharmaceutical composition has been manufactured by a process utilising intensive mixing in the form of co-milling and without utilising liquid at all wherein the molar ratio between said NSAID or pharmaceutically acceptable salt or prodrug thereof and said alkaline substance(s) or a derivative thereof ranges between 1:100 and 1:1.
In a still further aspect
A stable pharmaceutical composition for oral administration comprising:

- an NSAID or a pharmaceutically acceptable salt or prodrug thereof; and
- one or more amino acids or a derivative thereof;

wherein said pharmaceutical composition has been manufactured by a process comprising mixing said NSAID or pharmaceutically acceptable salt or prodrug thereof and said one or more amino acids or a derivative thereof and optionally one or more excipients by co-milling without adding a liquid, wherein the molar ratio between said NSAID or pharmaceutically acceptable salt or prodrug thereof and said amino acid(s) or a derivative thereof ranges between 1:100 and 1:1.
In one embodiment of this aspect the amino acid is histidine, lysine or arginine.
In one embodiment of both latter aspects , the NSAID is ampiroxicam, droxicam, lornoxicam, meloxicam, piroxicam, tenoxicam, ibuprofen or dexibuprofen or a pharmaceutically acceptable salt or prodrug thereof.

In one embodiment of both latter aspects, the NSAID is lornoxicam or a pharmaceutically acceptable salt or prodrug thereof.

In one embodiment of both latter aspects, the NSAID is lornoxicam.

In one embodiment of both latter aspects, the composition has an *in vitro* dissolution profile, when being subjected to dissolution test method using 0.1 N HCl equilibrated at 37 °C as the dissolution medium and USP paddle dissolution apparatus applied with a stirring rate of 50 rpm as the equipment, characterised in that at least 50% w/w of the active substance is present on dissolved form in the dissolution medium at the time point of 20 minutes after start of the dissolution testing.

*Examples*

The following non-limiting examples are meant to illustrate the present invention.

**EXAMPLES**

**Example 1:**

*Dissolution of the resulting particulate matter and oral dosage form*

*Dissolution method for testing the release of lornoxicam from a tablet*

*Dissolution method*

The following dissolution method aims at simulating the conditions in the stomach:

Apparatus: USP Dissolution Apparatus 2 equipped with paddles (as mentioned in USP 27 <711>)
Filters: Whatman GF/F glasfiber filters
Dissolution medium: 1300 ml of 0.1 N HCl with 2g/l of sodium chloride added (examples 2 and 3) or 1300 ml of 0.07 N HCl with 2g/l of sodium chloride added (examples 4 to 11)
Stirring rate: 50 rpm (examples 2 and 3) or 150 rpm (examples 4 to 11).
Temperature: 37°C $\pm$ 0.5°C
Sampling: Samples are taken every 5 minutes for a period of at least 60 minutes.
Quantification: The concentration of *lornoxicam* is determined in each sample using UV/Vis Spectrophotometer equipped with 10 mm cuvette and detection wavelength of 378nm. $E_{1\,cm}^{1\%}:587.0.$ Each sample was tested with n = 3.

**Example 2:**

*The effect of co-milling of lornoxicam and trisodium phosphate on the dissolution of lornoxicam in acid solution.*

*In the present example the co-milling was performed with a ball-mill having horizontally moving spheres.*

Ingredients:

| | | | | |
|---|---|---|---|---|
| 1. | Lornoxicam | | 8 | mg/tablet |
| 2. | Trisodium phosphate ($Na_3PO_4$) | | 78 | mg/tablet |
| 3. | Cellulose, microcrystalline | | 96 | mg/tablet |
| 4. | Calcium monohydrogen phosphate, anhydrous | | 110.4 | mg/tablet |
| 5. | Low substituted hydroxypropylcellulose | | 48 | mg/tablet |
| 6. | Binder (either a or b) | | 16 | mg tablet |
| | | a) Hydroxypropylcellulose (HPC-L-fine) | | |
| | | b) Vinylpyrrolidon-Vinylacetate | | |
| 7. | Calcium stearate | | 1.6 | mg/tablet |

(continued)

| Total core mass: | 358 | mg |
|---|---|---|

Batch size: approx. 750 g

The amount of *lornoxicam* and trisodium phosphate was in the molar relationship of 1:20.

Lornoxicam. (1) and trisodium phosphate (2) were co-milled for 5 minutes using a Fritsch Pulverisette (type 06.002.00; a ball mill with horizontally moving spheres). To the co-milled mixture of 1 and 2 was admixed further ingredients (3), (4), (5), (6) and (7) using an Erweka tumble mixer at 25 rpm for 5 min. The resulting mixture ("mixture of ingredients 1-7") was compressed into tablets using a Korsch 106 tabletting machine and 10.0 mm round standard concave punches.

Dissolution testing according to the method of Example 1 were conducted on the "mixture of ingredients 1-7" as well as on the resulting tablets.

**Table 2**

| Dissolution results for both the "mixture of ingredients 1-7" and corresponding tablets | | | |
|---|---|---|---|
| Type of binder | Type of product | % (w/w) dissolved lornoxicam at 20 min (n=3)* | % (w/w) dissolved lornoxicam at 20 min (n=3); corrected values |
| HPC | Powder mixture "mixture of ingredients 1-7" | 81 | 51 |
| | Tablet | 96 | 88 |
| VA 64 | Powder mixture "mixture of ingredients 1-7" | 77 | 54 |
| | Tablet | 92 | 82 |
| * At a later stage in the development process the dissolution method proved to be misleading by giving too high values. The corrected values are listed in the last column. | | | |

As can be derived from Table 2, fast dissolution of lornoxicam in an acidic solution is achieved upon applying co-milling of *lornoxicam* and trisodium phosphate in that more than 50% of the *lornoxicam* is dissolved from the "mixture of ingredients 1-7" at the time point of 20 minutes after starting the dissolution testing. Furthermore, it can be concluded that the process of compressing the "mixture of ingredients 1-7" into tablets results in even higher dissolution of *lornoxicam* at 20 minutes after starting the dissolution testing. It is our theory that the compacting process which may strengthen the contact between *lornoxicam* and trisodium phosphate causes this.

**Example 3:**

*The effect of co-milling of lornoxicam and sodium carbonate on the dissolution of lornoxicam in an acidic solution.*

*In the present example the co-milling was performed with a balt-mill having horizontally moving spheres.*

Ingredients:

| | | | |
|---|---|---|---|
| 1. | Lornoxicam | 8 | mg/tablet |
| 2. | Sodium carbonate decahydrate ($Na_2CO_3$, 10 $H_2O$)) | 136.2 | mg/tablet |
| 3. | Cellulose, microcrystalline | 96 | mg/tablet |
| 4. | Calcium monohydrogen phosphate, anhydrous | 110.4 | mg/tablet |
| 5. | Low substituted hydroxypropylcellulose | 48 | mg/tablet |
| 6. | Binder (either a or b) | 16 | mg tablet |
| | a. Hydroxypropylcellulose (HPC-L-fine) | | |
| | b. Vinylpyrrolidon-Vinylacetate | | |
| 7. | Calcium stearate | 1.6 | mg/tablet |

(continued)

| Total core mass: | 416.2 | mg |
|---|---|---|

Batch size was: Approx. 750 g

The amount of *lornoxicam* and sodium carbonate decahydrate is in a molar relationship of 1:20.

The "mixture of ingredients 1-7" as well as the corresponding tablets were made as described in example 2 and dissolution testing was carried out according to example 1.

The following dissolution results were obtained:

**Table 3**

| Dissolution results for both the "mixture of ingredients 1-7" and the corresponding tablets | | | |
|---|---|---|---|
| Type of binder | Type of product | % (w/w) dissolved lornoxicam at 20 min (n=3)* | % (w/w) dissolved lornoxicam at 20 min (n=3); corrected values |
| HPC | Powder mixture "mixture of ingredients 1-7" | 83 | 49 |
| | Tablet | 89 | 68 |
| VA 64 | Powder mixture "mixtures of ingredients 1-7" | 75 | 51 |
| | Tablet | 91 | 73 |
| *At a later stage in the development process the dissolution method proved to be misleading by giving too high values. The corrected values are listed In the last column. | | | |

The results are In accordance with the results obtained in example 2, thus showing that fast dissolution in an acidic solution is also achieved with co-milling of lornoxicam and sodium carbonate.

**Example 4:**

*The effect of co-milling of lornoxicam and arginine on the dissolution of lornoxicam in an acidic solution.*

*In the present example the co-milling is performed by means of a ball mill with horizontally moving spheres.*

Ingredients:

| | Ingredients | % (w/w) |
|---|---|---|
| 1 | Lornoxicam | 2.0 |
| 2 | Arginine | 18.7 |
| 3 | Cellulose microcrystalline, type 102 | 20.8 |
| 4 | Calcium monohydrogen phosphate, dihydrate $CaHPO_4$, $2H_2O$ | 47.8 |
| 5 | Low substituted hydroxypropyl cellulose | 10.4 |
| 6 | Calcium Stearate | 0.3 |
| | Tablet weight (mg) | 313 |
| | Particle size of the alkaline substance D(v;0.5) ($\mu$m) | 187$\mu$m |

The amount of *lornoxicam* and arginine is in a molar relationship of 1:20.

Tablets were made as described in example 2 and dissolution testing was carried out according to example 1.

Table 4. Dissolution of the obtained tablets

| Type of alkaline substance | % (w/w) dissolved lornoxicam at 20 minutes |
|---|---|
| Arginine | 78.0 |

As can be seen from table 4 when comparing with example 9 (prior process without the wet-granulation step), a significant increase in the amount dissolved at 20 min. can be achieved when co-milling *lornoxicam* and arginine by use of a ball mill with horizontally moving spheres.

**Example 5:**

*The effect of mechano fusion as the co-milling method on the dissolution of lornoxicam in an acidic solution.*

Ingredients:

| | Ingredients | Batch 170305 31 % (w/w) | Batch 170305 32 % (w/w) | Batch 170305 34 % (w/w) | Batch 170305 37 % (w/w) | Batch 170305 38 % (w/w) |
|---|---|---|---|---|---|---|
| 1 | Lornoxicam | 1.7 | 1.7 | 2.8 | 2.6 | 2.3 |
| 2 | Sodium carbonate, $Na_2CO_3$ | | | 17.9 | | |
| 2 | Trisodium phosphate $Na_3PO_4$ | 19.0 | 19.0 | | | |
| 2 | Arginine | | | | 18.1 | |
| 2 | Lysine | | | | | 18.4 |
| 3 | Cellulose microcrystalline, type 102 | 20.8 | 20.8 | 20.8 | 20.8 | 20.8 |
| 4 | Calcium monohydrogen phosphate, dihydrate $CaHPO_4, 2H_2O$ | 47.8 | 47.8 | 47.8 | 47.8 | 47.8 |
| 5 | Low substituted hydroxypropyl cellulose | 10.4 | 10.4 | 10.4 | 10.4 | 10.4 |
| 6 | Calcium Stearate | 0.3 | 0.3 | 0.3 | 0.3 | 0.3 |
| | Tablet weight (mg) | 314 | 306 | 278 | 298 | 244 |
| | Mean particle size of the alkaline substance D(v;0.5) ($\mu$m) | 203 | 40 | 48 | 68 | 46 |

The amount of lornoxicam and the alkaline substance is in a molar relationship of app. 1:20

Prior to the mechano fusion the alkaline substance (2) was milled by means of a Alpine® AS spiral jet mill from Hosokawa Alpine. The resulting mean particle size was determined and listed in the table above.

Co-milling of the alkaline substance (2) and lornoxicam (1) was performed with a AMS-LAB mechano fusion unit from Hosokawa Alpine. The parameters were as follows:

- Time: 3 - 30 min
- Rotor speed: 1300 - 1500
- Temp: 20 - 45 °C

The rest of the Ingredients listed (3) to (6) were admixed and tablets with a diameter of 10 mm were compressed. Dissolution testing according to example 1 was carried out.

Table 5. Dissolution results of resulting tablets

| Type of alkaline substance | Batch no. | % (w/w) dissolved lornoxicam at 20 minutes | Mean particle size D(v;0.5) ($\mu$m) |
|---|---|---|---|
| Trisodium phosphate, $Na_3PO_4$ | 17030531 | 57.0 | 203 |
| Trisodium phosphate, $Na_3PO_4$ | 17030532 | 67.2 | 40 |
| Sodium carbonate, $Na_2CO_3$ | 17030534 | 63.2 | |
| Arginine | 17030537 | 62.3 | |
| Lysine | 17030538 | 80.1 | |

From table 5 is seen that co-milling by mechano fusion of lornoxicam and the alkaline substance lysine results in a significant increase in the amount dissolved at 20 minutes when comparing with example 9 (prior process without the wet-granulation step). When using arginine, trisodium phosphate or sodium carbonate a positive impact on the dissolution can also be seen. Furthermore, the results illustrate that when using mechano fusion a small mean particle size of the alkaline substance has a positive impact on the dissolution. This is illustrated by the results achieved with co-milled mixtures containing trisodium phosphate where the batch having the small mean particle size of the alkaline substance has a faster dissolution than the one having the relatively large mean particle size.

**Example 6:**

*The effect of using roller compaction as the method of co-milling on the dissolution of lornoxicam In an acidic solution.*

Ingredients:

| | Ingredients | Batch 13050534 % (w/w) | Batch 13050535 % (w/w) | Batch 13050543 % (w/w) | Batch 130605431 % (w/w) | Batch 13060534 % (w/w) |
|---|---|---|---|---|---|---|
| 1 | Lornoxicam | 1.7 | 1.7 | 2.3 | 2.3 | 2.3 |
| 2 | Trisodium phosphate $Na_3PO_4$ | 19.0 | 19.0 | | | |
| 2 | Lysine | | | 18.4 | 18.4 | 18.4 |
| 3 | Cellulose microcrystalline, type 102 | 20.8 | 20.8 | 20.8 | 20.8 | 20.8 |
| 4 | Calcium monohydrogen phosphate, dihydrate $CaHPO_4, 2H_2O$ | 47.8 | 47.8 | 47.8 | 47.8 | 47.8 |
| 5 | Low substituted hydroxypropyl cellulose | 10.4 | 10.4 | 10.4 | 10.4 | 10.4 |

(continued)

|  | Ingredients | Batch 13050534 % (w/w) | Batch 13050535 % (w/w) | Batch 13050543 % (w/w) | Batch 130605431 % (w/w) | Batch 13060534 % (w/w) |
|---|---|---|---|---|---|---|
| 6 | Calcium Stearate | 0.3 | 0.3 | 0.3 | 0.3 | 0.3 |
|  | Tablet weight (mg) | 336 | 318 | 261 | 314 324 |  |
|  | Mean particle size of the alkaline substance D(v;0.5) (μm) | 24μm | 203μm | 158μm | 26μm | 15μm |

The amount of lornoxicam and the alkaline substance is in a molar relationship of approx. 1:20

Prior to roller compaction the alkaline substance (2) used in batches 13050534 and 13060531 was milled using a Fritz Pulverlsette (type 14.702). The resulting mean particle sizes are listed In the table above.

Roller compaction of the alkaline substance (2) and lornoxicam (1) was carried out using a Minipactor® from Gerteis Maschinen + Processengineering AG. The parameters were as follows:

Compaction force:   8 - 12 kN/cm
Rpm:   2
Sieve size:   1.0 - 1.5 mm
Gab size   2,5 mm

The rest of the ingredients listed above (3) to (6) were admixed and tablets having a diameter of 10 mm were compressed. Dissolution testing according to example 1 was carried out.

Table 6. Dissolution of resulting tablets

| Type of alkaline substance | Batch no. | % (w/w) dissolved lornoxicam at 20 minutes |
|---|---|---|
| Trisodium phosphate $Na_3PO_4$ | 13050534 | 59.2 |
| Trisodium phosphate $Na_3PO_4$ | 13050535 | 57.6 |
| Lysine | 13050543 | 89,5 |
| Lysine | 13060531 | 79.3 |
| lysine | 13060534 | 82.3 |

From table 6 is seen that by performing co-milling by means of roller compaction the use of lysine as alkaline substance leads to a significant increase in amount dissolved at 20 min when comparing with the results of example 9 (prior process without the wet-granulation step). When using trisodium phosphate as the alkaline substance a positive impact on the dissolution can also be seen. The difference in mean particle size of the alkaline substance has no major impact on dissolution when roller compaction is used as the method of co-milling.

**Example 7:**

*The effect on the dissolution of lornoxicam in tablet where the alkaline substance was micronized prior to simple admixing followed by compression into a tablet.*

Ingredients

|  | Ingredients | Batch 02060531 % (w/w) | Batch 02060532 % (w/w) |
|---|---|---|---|
| 1 | Lornoxicam | 2.3 | 1.2 |

(continued)

| | | Ingredients | Batch 02060531 % (w/w) | Batch 02060532 % (w/w) |
|---|---|---|---|---|
| | 2 | Lysine | 18.4 | 19.5 |
| | 3 | Cellulose microcrystalline, type 102 | 20.8 | 20.8 |
| | 4 | Calcium monohydrogen phosphate, dihydrate, $CaHPO_4, 2H_2O$ | 47.8 | 47.8 |
| | 5 | Low substituted hydroxypropyl cellulose | 10.4 | 10.4 |
| | 6 | Calcium Stearate | 0.3 | 0.3 |
| | | Tablet weight (mg) | 300 | 429 |
| | | Mean particle size of the alkaline substance D(v; 0.5) ($\mu$m) | 5.0 | 5.0 |

The amount of lornoxicam and the alkaline substance is in a molar relationship of app. 1:20 for batch 02060531 and approx. 1:40 for batch 02060532.

The alkaline substance (2) was micronized by use of a Alpine® AS spiral jet mill from Hosokawa Alpine.

The alkaline substance (2) and the lornoxicam (1) were mixed by use of a blender (with propeller-like blades at the bottom).

Thereafter, the rest of the ingredients (3) to (6) were admixed by use of a tumble mixer and tablets having a diameter of 10 mm were compressed. Dissolution testing according to example 1 was carried out.

Table 7. Dissolution results of resulting tablets

| Type of alkaline substance | Batch no. | % (w/w) dissolved lornoxicam at 20 minutes |
|---|---|---|
| Lysine | 02060531 | 82,0 |
| Lysine | 02060532 | 82,9 |

From table 7 is seen that blending of micronized lysine and lornoxicam leads to a significant increase in the amount dissolved at 20 min when compared to example 9 (prior process without the wet-granulation step). An increase in the molar relationship of lysine, changing the ratio between lornoxicam and lysine from 1:20 to 1:40 does not have any major impact on the dissolution.

**Example 8:**

*The effect on the dissolution of lornoxicam from tablets where the alkaline substance was subjected to milling prior to compressing the mixture of ingredients into tablets. The dissolution is performed in an acidic solution.*

Ingredients

| | Ingredients | Milled mixture % (w/w) |
|---|---|---|
| 1 | Lornoxicam | 1.7 |
| 2 | Trisodium phosphate, dried * $Na_3PO_4$ | 19.0 |
| 3 | Cellulose microcrystalline, type 102 | 20.8 |
| 3 | Cellulose microcrystalline, type 101 | |
| 4 | Calcium monohydrogen phosphate dihydrate $CaHPO_4, 2H_2O$ | 47.8 |
| 4 | Calcium monohydrogen phosphate anhydrous $CaHPO_4$ | |
| 5 | Low substituted hydroxypropyl cellulose | 10.4 |

(continued)

| | Ingredients | Milled mixture % (w/w) |
|---|---|---|
| 5 | Hydroxypropylcellulose | |
| 6 | Calcium stearate | 0.3 |
| | Tablet weight (mg) | 311 |
| | Mean particle size of alkaline substance D(v;0:5) ($\mu$m) | 79 |
| *): $Na_3PO_4$, 12 $H_2O$ were dried to a content of water less than approx. 2% (w/w). The amount of lornoxicam and the alkaline substance is in a molar relationship of approx. 1:20. | | |

The dried trisodium phosphate (2) was milled using a Fritsch Pulverisette (type 06.002.00; a ball mill with horizontally moving spheres) and mixed with lornoxicam (1) by hand. Thereafter, the rest of the excipients (3) to (6) were admixed in a tumble mixer. From the mixture of ingredients (1) to (6) were compressed tablets having a diameter of 10 mm. Dissolution testing according to example 1 was carried out.

Table 8. Dissolution results of the obtained tablets

| Alkaline substance | % (w/w) dissolved lornoxicam at 20 minutes |
|---|---|
| Trisodium phosphate, dried * $No_3PO_4$ | 53.1 |
| *): $Na_3PO_4$, 12 $H_2O$ were dried to a content of water less than app. 2% (w/w). | |

From table 8 is seen that prior milling of the alkaline material, trisodium phosphate, before compressing the mixture of ingredients into tablets, improves the amount dissolved at 20 minutes when compared to example 9.

**Example 9:**

*The impact of abandoning wet-granulation in the manufacturing process while otherwise performed according to* EP 1109534,

Ingredients

| | Ingredients | % (w/w) |
|---|---|---|
| 1 | Lornoxicam | 2.5 |
| 2 | Sodium bicarbonate $NaHCO_3$ | 12.5 |
| 3 | Cellulose microcrystalline, type 101 | 30.0 |
| 4 | Calcium monohydrogen phosphate, anhydrous $CaHPO_4$ | 34.5 |
| 5 | Low substituted hydroxylpropyl cellulose | 15.0 |
| 5 | Hydroxpropyl cellulose | 5.0 |
| 6 | Calcium Stearate | 0.5 |
| | Tablet weight (mg) | 320 |

The amount of lornoxicam and the alkaline substance is in a molar relationship of approx. 1:20.
The ingredients (2) to (5) were mixed using a Diosna high shear mixer. Thereafter, the lornoxicam (1) was admixed lege artis using a planetary mixer. Finally the calcium stearate (6) was admixed using a high shear mixer.
Tablets were compressed using a 10 mm round standard concave tablet design. Dissolution testing according to example 1 was carried out

Table 9. Dissolution results of the obtained tablets

| Alkaline substance | % (w/w) dissolved lornoxicam at 20 minutes |
| --- | --- |
| Sodium bicarbonate, $NaHCO_3$ | 31.3 |

From table 9 is seen that the dissolution result of the obtained tablets where the wet-granulation step is left out is below 50%. The obtained dissolution is clearly not satisfactory.

**Example 10:**

*The impact of co-milling of lornoxicam and the alkaline substance on the chemical stability of lornoxicam.*

*The co-milling is performed with ball milling with horizontally moving spheres.*
To compare the chemical stability of compositions produced according to the invention with compositions produced by means of wet-granulation, the following batches were provided:
Tablets according to the invention, batch nos: 17110431 and 17110432 Tablets manufactured according to the invention comprising co-milling of the alkaline substance and lornoxicam were manufactured according to examples 2 and 3 with the below described variations:

Ingredients:

| | Ingredients | Wet granulation Batch 10225671 % (w/w) | Co-milling, Batch 17110431 % (w/w) | Co-milling Batch 17110432 % (w/w) |
| --- | --- | --- | --- | --- |
| 1 | Lornoxicam | 2.5 | 2.4 | 1.7 |
| 2 | Sodium bicarbonate $NaHCO_3$ | 12.5 | | |
| 2 | Sodium carbonate, dried * $Na_2CO_3$ | | 15.6 | |
| 2 | Trisodium phosphate, dried ** $Na_3PO_4$ | | | 19.0 |
| 3 | Cellulose microcrystalline, type 101 | 30.0 | | |
| 3 | Cellulose microcrystalline, type 102 | | 21.5 | 20.7 |
| 4 | Calcium monohydrogen phosphate, anhydrous $CaHPO_4$ | 34.5 | | |
| 4 | Calcium monohydrogen phosphate dihydrate $CaHPO_4, 2H_2O$ | | 49.4 | 47.8 |
| 5 | Low substituted hydroxypropyl cellulose | 15.0 | 10.7 | 10.4 |
| 5 | Hydroxypropylcellulose | 5.0 | | |
| 6 | Calcium Stearate | 0.5 | 0.4 | 0.4 |

(continued)

|  | Ingredients | Wet granulation Batch 10225671 % (w/w) | Co-milling, Batch 17110431 % (w/w) | Co-milling Batch 17110432 % (w/w) |
|---|---|---|---|---|
|  | Tablet weight (mg) | 320 | 329 | 351 |

*): $Na_2CO_3$, 10 $H_2O$ were dried to a content of water less than app. 2% (w/w).
**): $Na_3PO_4$, 12 $H_2O$ were dried to a content of water less than app. 2% (w/w)

The amount of lornoxicam and the alkaline substance is in a molar relationship of approx. 1:20.

Co-milling of lornoxicam (1) together with the alkaline substance (2) was carried out for 10 min by use of a Fritsch Pulverisette (type 06.002.00; a ball mill with horizontally moving spheres). The rest of the ingredients listed above (3) to (6) were admixed and tablets having a diameter of 10 mm were compressed using a standard concave punch design. From the mixture of ingredients (1) to (6) were compressed tablets using a 10 mm round standard concave punch design.

Tablet produced with wet-granulation, batch no: 10225671

The tablets were manufactured according to EP 1109534: The tablets based on wet granulation were compressed using a 9.5 mm round standard concave punch design. Furthermore, tablets based on wet granulation were coated as also described in EP 1109534.

Stability program

A stability program was performed, including the following batches:

Batch no: 10225671, Xefo Rapid, wet-granulation
Batch no: 17110431, co-milling
Batch no: 17110432, cho-mining

The co-milled batches were compared to a batch, which was produced by means of wet-granulation. The degradation product of lornoxicam, HN-10004 was chosen as stability indicating parameter.

All three batches were packed and stored in alu-alu bags.

Condition of storage:

25°C/60% RH for six months.
30°C/65% RH for six months
40°C/75% RH for six months

Conclusion

At all testpoints the amount of HN-10004 in the batch nos. 17110431 and 17110432 (co-milled) was lower than in batch no. 10225671 (wet-granulated).

At the starting point of the test period, the water-content of the tablets were determined and found to be on the same level. At all the test points, the stability of the two co-milled batches were superior to a significant extent over the wet-granulated batch, despite the fact that the wet-granulated batch has a lower water-content.

From the results of the stability testing it is concluded that the use of co-milling leads to a composition having a significantly slower development in the formation of critical degradation products.

Results

At the starting point the water-content of the tablets were determined by Loss on Drying (LOD) (30 min, 70 °C). The results are shown in table 10 below:

Table 10. Water content in the resulting tablets.

| Batch no | Water content (LOD) % (w/w) |
|---|---|
| 10225671 | 1.2 |
| 17110431 | 1.5 |
| 17110432 | 1.6 |

| Storage time (months) | 25°C/60% RH | | |
|---|---|---|---|
| | Batch no: 10225671 HN-10004 in % (w/w) of lornoxicam | Batch no: 17110431 HN-10004 in % (w/w) of lornoxicam | Batch no: 17110432 HN-10004 in % (w/w) of lornoxicam |
| 0 | 0.073 | 0.012 | 0.010 |
| 1.5 | 0.11 | 0.012 | 0.032 |
| 3 | 0.13 | 0.024 | 0.013 |
| 4.5 | 0.15 | 0.033 | 0.030 |
| 6 | 0.17 | 0.036 | 0.035 |

| Storage time (months) | 30°C/65% RH | | |
|---|---|---|---|
| | Batch no: 10225671 HN-10004 in % (w/w) of lornoxicam | Batch no: 17110431 HN-10004 in % (w/w) of lornoxicam | Batch no: 17110432 HN-10004 in % (w/w) of lornoxicam |
| 0 | 0.073 | 0.012 | 0.010 |
| 1.5 | 0.047 | 0.015 | 0.014 |
| 3 | 0.17 | 0.037 | 0.024 |
| 4.5 | 0.18 | 0.047 | 0.044 |
| 6 | 0.22 | 0.051 | 0.053 |

| Storage time (months) | 40°C/75% RH | | |
|---|---|---|---|
| | Batch no: 10225671 HN-10004 in % (w/w) of lornoxicam | Batch no: 17110431 HN-10004 in % (w/w) of lornoxicam | Batch no: 17110432 HN-10004 in % (w/w) of lornoxicam |
| 0 | 0.073 | 0.012 | 0.010 |
| 3 | 0.22 | 0.072 | 0.090 |
| 6 | 0.22 | 0.12 | 0.11 |

Method of analysis (HPLC):

Stationary phase: ODS, 5 μm, 100 x 2.1 mm.

Column temperature: 35°C

Mobile phase: Solvent A: Dissolve 50.0 g ammonium acetate in 5000.0 mL Milli-Q-water, and add 8.0 mL tetrabutyl ammonium hydroxide (1.5M in water) and 22.0 mL methanol.

Solvent B: Add 1.00 mL octylamine to 1000.00 mL acetonitrile.

Gradient:

| Time (min.) | Flow (mL/min.) | Solvent A % | Solvent B % |
|---|---|---|---|
| 0 | 0.5 | 97 | 3 |
| 32 | 0.5 | 70 | 30 |
| 36 | 0.5 | 70 | 30 |
| 41 | 0.5 | 97 | 3 |
| 50 | 0.5 | 97 | 3 |

Autosampler temperature: 20°C

Injection volume: 20 $\mu$L

Detection: 280 nm

Runtime: 50 minutes

The relative retention time for HN-10004, calculated with respect to the principal peak, is approximately 0.77 and the relative response factor is 0.8.

**Example 11:**

*The effect of co-milling on the dissolution of lornoxicam. In the present example the co-milling is performed by means of ball milling with vertically moving spheres.*

Ingredients

| | Ingredients | Batch 07020533 % (w/w) | Batch 07020534 % (w/w) | Batch 19040531 % (w/w) | Batch 03050532 % (w/w) |
|---|---|---|---|---|---|
| 1 | Lornoxicam | 2.3 | 1.7 | 1.2 | 1.2 |
| 2 | Trisodium phosphate, dried *) $Na_3PO_4$ | | 19.0 | | |
| 2 | Lysine | 18.4 | | 19.5 | 19.5 |
| 3 | Cellulose microcrystalline, type 102 | 20.8 | 20.8 | 20.8 | 20.8 |
| 4 | Calcium monohydrogen phosphate, dihydrate $CaHPO_4, 2H_2O$ | 47.8 | 47.8 | 47.8 | 47.8 |
| 5 | Low substituted hydroxypropyl cellulose | 10.4 | 10.4 | 10.4 | 10.4 |
| 6 | Calcium Stearate | 0.3 | 0.3 | 0.3 | 0.3 |
| | Tablet weight (mg) | 238 | 318 | 277 | 420 |

(continued)

|  | Ingredients | Batch 07020533 % (w/w) | Batch 07020534 % (w/w) | Batch 19040531 % (w/w) | Batch 03050532 % (w/w) |
|---|---|---|---|---|---|
|  | Mean particle size of alkaline substance D(v;0.5) ($\mu$m) | 158 | - | 158 | 26 |
| *): Na$_3$PO$_4$, 12 H$_2$O were dried to a content of water less than app. 2% (w/w) | | | | | |

The amount of lornoxicam and the alkaline substance is in a molar relationship of approx. 1:20 for batch 07020533, 07020534 and approx. 1:40 for batch 19040531 and 03050532. Lornoxicam (1) and lysine (2) were co-milled using a Struers ball mill for 4 - 10 hours and 250 - 400 rpm followed by a sieving through a 700$\mu$m mesh. Thereafter, the rest of the excipients (3) to (6) were admixed and tablets were compressed using a round 10 mm standard concave tablet design. Dissolution testing according to example 1 was carried out.

Table 11. Dissolution results of the obtained tablets.

| Alkaline substance | Batch no. | % (w/w) dissolved lornoxicam at 20 min. |
|---|---|---|
| Lysine | 07020533 | 80.8 |
| Trisodium phosphate | 07020534 | 66.2 |
| Lysine | 19040531 | 85.6 |
| Lysine | 03050532 | 82.7 |

From table 11 can be seen that the use of a ball mill with vertically moving spheres results in a significantly improved amount of lornoxicam dissolved at 20 min when compared to  example 9 (prior process without the wet-granulation step). An increase in the molar relationship between lornoxicam and lysine from 1:20 to 1:40 does not have any major impact on the dissolution.

**Example 12:**

Examples of compositions to be manufactured by co-milling by means of roller compaction of lornoxicam, the alkaline substance and selected other excipients followed by admixture of the rest of the excipients and compressing into tablets.

Ingredients

|  |  | Ingredients | % (w/w) | % (w/w) | % (w/w) | % (w/w) | % (w/w) | % (w/w) |
|---|---|---|---|---|---|---|---|---|
| 1 | | Lornoxicam | 1-3 | 1-3 | 1-3 | 1-3 | 1-3 | 1-3 |
| 2 | | Lysine | 9-20 | 9-20 | 9-20 | 9-20 | 9-20 | 9-20 |
|  | | Molar relationship, lornoxicam:lysine | 1:10-1:40 | 1:10-1:40 | 1:10-1:40 | 1:10-1:40 | 1:10-1:40 | 1:10-1:40 |
| 3 | | Calcium monohydrogen phosphate, anhydrous CaHPO$_3$ | 10-50 | | | | | |
| 3 | | Trisodium phosphate Na$_3$PO$_4$ | | 10-50 | | | | |
| 3 | | Calcium Carbonate CaCO$_3$ | | | 10-50 | | | |
| 3 | | Magnesium Aluminium Silicate | | | | 10-50 | | |

(continued)

| | | Ingredients | % (w/w) | % (w/w) | % (w/w) | % (w/w) | % (w/w) | % (w/w) |
|---|---|---|---|---|---|---|---|---|
| | 3 | Magnesium oxide | | | | | 10-50 | |
| | 3 | Calcium sulphate dihydrate $CaSO_4, 2H_2O$ | | | | | | 10-50 |
| | 4 | Sorbitol | | | 5 - 15 | | | |
| | 5 | Talc | 0 - 10 | 0 - 10 | 0 - 10 | 0-10 | 0 - 10 | 0 - 10 |
| | 6 | Cellulose microcrystalline, type 102 | 15-25 | 15 - 25 | 15 - 25 | 15 - 25 | 15 - 25 | 15-25 |
| | 7 | Low substituted hydroxypropyl cellulose | 5 - 15 | 5 - 15 | 5 - 15 | 5 - 15 | 5-15 | 5 - 15 |
| | 8 | Calcium Stearate | 0.2-1.0 | 0.2-1.0 | 0.2-1.0 | 0.2-1.0 | 0.2-1.0 | 0.2-1.0 |

The ingredients (1) to (2) are premixed, sieved and mixed. The mean particle size of (2) is ranging between a D(v;0.5) of 5 μm to 160μm. Thereafter, ingredient (5) is admixed following by admixture of (3) and (4). This mixture is co-milled by use of methods like roller compaction, ball milling (both horizontally and vertically moving spheres) or mixing in a blender. Thereafter the rest of the ingredients (6) to (8) are combined with the mixture of ingredients (1) to (5) by admixing. The combined mixture of the ingredients (1) to (8) is compressed into tablets.

The individual amounts are adjusted so that each composition contains 4-12 mg lornoxicam, the molar ratio of lornoxicam to alkaline substance is in the range of 1:10 to 1:40 and so that the total amount of ingredients does not exceed 100%.

Tablets having a diameter of 10mm are compressed by use of punch design of a round standard concave. The tablets can be coated afterwards in order to obtain coloured or white or moisture protected tablets as described in EP 1109534.

**Claims**

1. A process for manufacturing an oral dosage form comprising an active drug substance having a fast dissolution in gastric fluid; said process comprising the steps of:

    a) providing the active drug substance;
    b) providing one or more alkaline substance(s); and
    c) mixing said active drug substance and said alkaline substance(s) and optionally one or more excipients by co-milling without adding a liquid, and optionally
    d) admixing one or more pharmaceutically acceptable excipients, and optionally
    e) compressing said mixture of c) or d) into a tablet;

    wherein the active drug substance has a solubility at room temperature of less than 0.1% w/v in 0.1 N hydrochloric acid or has a pKa value less than 5.5; and wherein the molar ratio between said active drug substance and said alkaline substance(s) ranges between 1:100 and 1:1.

2. The process according to claim 1, wherein the co-milling step (c) is applied only to the active drug substance and the alkaline substance(s).

3. The process according to claim 1 or 2 wherein said process is undertaken under dry conditions excluding the use of liquid.

4. The process according to any one of claims 1 to 3, wherein the molar ratio of the active drug substance and the alkaline substance(s) is between 1:100 and 1:10.

5. The process according to any one of claims 1 to 4, wherein the molar ratio of the active drug substance and the alkaline substance(s) is/are in the range of 1:10 to 1:40, and wherein said active drug substance is lornoxicam.

6. The process according to any one of claims 1 to 5, wherein the alkaline substance(s) is/are water soluble as **characterised by** that 1 part of the alkaline substance can be dissolved in a maximum of 100 parts of water.

7. The process according to any one of claims 1 to 6, wherein the alkaline substance(s) is/are a salt of an organic acid, a salt of an inorganic acid, an organic amine or an amino acid or a derivative thereof.

8. The process according to any one of claims 1 to 7, wherein the alkaline substance(s) is/are an amino acid or a derivative thereof.

9. The process according to claim 8, wherein the amino acid or a derivative thereof is lysine, arginine or histidine.

10. The process according to claim 7, wherein the organic acid and the inorganic acid have a pKa in the range of 4-14.

11. The process according to claim 7, wherein the alkaline substance(s) is/are a salt of an inorganic acid selected from carbonic acid or phosphoric acid.

12. The process according to any one of claims 1 to 11, wherein the active drug substance is an NSAID or a pharmaceutically acceptable salt or prodrug thereof.

13. The process according to claim 12, wherein the NSAID is a thiazinecarboxamide or a pharmaceutically acceptable salt or a prodrug thereof.

14. The process according to claim 12, wherein the NSAID is ampiroxicam, droxicam, lornoxicam, meloxicam, piroxicam, tolfenamic acid or tenoxicam or a pharmaceutically acceptable salt or prodrug thereof.

15. The process according to any one of claims 1 to 14, wherein said co-milling is provided by a roller compactor.

16. The process according to any one of claims 1 to 15, wherein the co-milling process is carried out using a Ball Mill.

17. A pharmaceutical composition obtainable by the process as defined in any one of claims 1 to 16.

18. The pharmaceutical composition according to claim 17 comprising:

    - an NSAID; and
    - an amino acid or a derivative thereof.

19. The pharmaceutical composition according to claim 17, wherein

    - the active drug substance is ampiroxicam, droxicam, lornoxicam, meloxicam, piroxicam, tenoxicam, bromazepam, ibuprofen, tolfenamic acid or dexibuprofen or a pharmaceutically acceptable salt or prodrug thereof; and
    - the alkaline substance is histidine, lysine or arginine.

20. The pharmaceutical composition according to claim 17, wherein

    - the active drug substance is lornoxicam or a pharmaceutically acceptable salt or prodrug thereof; and
    - the alkaline substance is histidine, lysine or arginine.

21. The pharmaceutical composition according to claim 17, wherein

    - the active drug substance is lornoxicam; and
    - the alkaline substance is histidine, lysine or arginine.

22. A stable pharmaceutical composition for oral administration comprising:

    - an NSAID or a pharmaceutically acceptable salt or prodrug thereof;
    - one or more alkaline substances selected from a salt containing an anion selected from $CO_3^{2-}$, $HPO_4^{2-}$, $PO_4^{3-}$ and a cation selected from $Na^+$ and $K^+$;

- and a binder in the form of a hydrophilic polymer;

wherein said pharmaceutical composition has been manufactured by a process utilising intensive mixing in the form of co-milling and without utilising liquid at all wherein the molar ratio between said NSAID or pharmaceutically acceptable salt or prodrug thereof and said alkaline substance(s) or a derivative thereof ranges between 1:100 and 1:1.

23. A stable pharmaceutical composition for oral administration comprising:

- an NSAID or a pharmaceutically acceptable salt or prodrug thereof; and
- one or more amino acids or a derivative thereof;

wherein said pharmaceutical composition has been manufactured by a process comprising mixing said NSAID or pharmaceutically acceptable salt or prodrug thereof and said one or more amino acids or a derivative thereof and optionally one or more excipients by co-milling without adding a liquid, wherein the molar ratio between said NSAID or pharmaceutically acceptable salt or prodrug thereof and said amino acid(s) or a derivative thereof ranges between 1:100 and 1:1.

24. The pharmaceutical composition according to claim 23, wherein the amino acid or a derivative thereof is histidine, lysine or arginine.

25. The pharmaceutical composition according to claims 22 or 23, wherein the NSAID is ampiroxicam, droxicam, lornoxicam, meloxicam, piroxicam, tenoxicam, ibuprofen or dexibuprofen or a pharmaceutically acceptable salt or prodrug thereof.

26. The pharmaceutical composition according to claims 22 or 23, wherein the NSAID is lornoxicam or a pharmaceutically acceptable salt or prodrug thereof.

27. The pharmaceutical composition according to claim 23, wherein the NSAID is lornoxicam and wherein the amino acid or a derivative thereof is lysine.

28. The pharmaceutical composition according to claim 23, wherein the NSAID is lornoxicam and wherein the amino acid or a derivative thereof is arginine.

29. The pharmaceutical composition according to any one of claims 17-28; wherein the composition comprises an opioid or triptan in combination with lornoxicam.

30. The pharmaceutical composition according to any one of claims 17-29 or the process according to any one of claims 1-16, wherein the NSAID or active drug substance is lornoxicam.

31. The pharmaceutical composition according to any one of claims 17-30 or the process according to any one of claims 1-16, wherein the composition or each oral dosage form comprises 4-12 mg lornoxicam.

32. The pharmaceutical composition according to any one of claims 22-31 or the oral dosage form according to any one of claims 1-16, wherein said composition or oral dosage form is in the form of a compressed tablet.

33. The pharmaceutical composition according to any one of claims 17-32 or the process according to any one of claims 1-16, wherein the molar ratio of lornoxicam and the alkaline substance(s) is/are between 1:100 and 1:10.

**Patentansprüche**

1. Verfahren zur Herstellung einer oralen Darreichungsform, umfassend einen Arzneimittelwirkstoff, der sich schnell im Magensaft löst; wobei das Verfahren folgende Schritte umfasst:

a) Bereitstellen des Arzneimittelwirkstoffs;
b) Bereitstellen einer oder mehrerer alkalischer Substanzen; und
c) Mischen des Arzneimittelwirkstoffs und der alkalischen Substanz(en) und fakultativ eines oder mehrerer Hilfsstoffe durch gemeinsames Vermahlen ohne die Zugabe einer Flüssigkeit und fakultativ
d) Zumischen eines oder mehrerer pharmazeutisch unbedenklicher Hilfsstoffe und fakultativ
e) Komprimieren der Mischung aus c) oder d) zu einer Tablette;

wobei der Arzneimittelwirkstoff bei Raumtemperatur eine Löslichkeit von weniger als 0,1 Gew./Vol.-% in 0,1 N Salzsäure oder einen pKa-Wert von weniger als 5,5 aufweist; und
wobei das Molverhältnis zwischen dem Arzneimittelwirkstoff und der/den alkalischen Substanz(en) im Bereich von 1:100 bis 1:1 liegt.

2. Verfahren nach Anspruch 1, wobei der Schritt (c) des gemeinsamen Vermischens nur mit dem Arzneimittelwirkstoff und der/den alkalischen Substanz(en) durchgeführt wird.

3. Verfahren nach Anspruch 1 oder 2, wobei das Verfahren unter trockenen Bedingungen unter Ausschluss der Verwendung von Flüssigkeit durchgeführt wird.

4. Verfahren nach einem der Ansprüche 1 bis 3, wobei das Molverhältnis zwischen dem Arzneimittelwirkstoff und der/den alkalischen Substanz(en) im Bereich von 1:100 bis 1:10 liegt.

5. Verfahren nach einem der Ansprüche 1 bis 4, wobei das Molverhältnis zwischen dem Arzneimittelwirkstoff und der/den alkalischen Substanz(en) im Bereich von 1:10 bis 1:40 liegt und wobei der Arzneimittelwirkstoff Lornoxicam ist.

6. Verfahren nach einem der Ansprüche 1 bis 5, wobei die alkalische(n) Substanz(en) wasserlöslich ist/sind, **dadurch gekennzeichnet, dass** 1 Teil der alkalischen Substanz in höchstens 100 Teilen Wasser gelöst werden kann.

7. Verfahren nach einem der Ansprüche 1 bis 6, wobei die alkalische(n) Substanz(en) ein Salz einer organischen Säure, ein Salz einer anorganischen Säure, ein organisches Amin oder eine Aminosäure oder ein Derivat davon ist/sind.

8. Verfahren nach einem der Ansprüche 1 bis 7, wobei die alkalische(n) Substanz(en) eine Aminosäure oder ein Derivat davon ist/sind.

9. Verfahren nach Anspruch 8, wobei die Aminosäure oder ein Derivat davon Lysin, Arginin oder Histidin ist.

10. Verfahren nach Anspruch 7, wobei die organische Säuren und die anorganische Säure einen pKa-Wert im Bereich von 4 bis 14 aufweisen.

11. Verfahren nach Anspruch 7, wobei die alkalische(n) Substanz(e) ein Salz einer anorganischen Säure ist/sind, ausgewählt aus Kohlensäure oder Phosphorsäure.

12. Verfahren nach einem der Ansprüche 1 bis 11, wobei der Arzneimittelwirkstoff ein NSAID oder ein pharmazeutisch unbedenkliches Salz oder Prodrug davon ist.

13. Verfahren nach Anspruch 12, wobei das NSAID ein Thiazincarboxamid oder ein pharmazeutisch unbedenkliches Salz oder Prodrug davon ist.

14. Verfahren nach Anspruch 12, wobei das NSAID Ampiroxicam, Droxicam, Lornoxicam, Meloxicam, Piroxicam, Tolfenaminsäure oder Tenoxicam oder ein pharmazeutisch unbedenkliches Salz oder Prodrug davon ist.

15. Verfahren nach einem der Ansprüche 1 bis 14, wobei das gemeinsame Vermahlen durch einen Walzenkompaktor bereitgestellt wird.

16. Verfahren nach einem der Ansprüche 1 bis 15, wobei das gemeinsame Vermahlen mit einer Kugelmühle durchgeführt wird.

17. Pharmazeutische Zusammensetzung, die durch das in einem der Ansprüche 1 bis 16 definierte Verfahren erhältlich ist.

18. Pharmazeutische Zusammensetzung nach Anspruch 17, umfassend:

   - ein NSAID; und
   - eine Aminosäure oder ein Derivat davon.

19. Pharmazeutische Zusammensetzung nach Anspruch 17, wobei:

   - der Arzneimittelwirkstoff Ampiroxicam, Droxicam, Lornoxicam, Meloxicam, Piroxicam, Tenoxicam, Bromazepam, Ibuprofen, Tolfenaminsäure oder Dexibuprofen oder ein pharmazeutisch unbedenkliches Salz oder Prodrug davon ist; und
   - die alkalische Substanz Histidin, Lysin oder Arginin ist.

20. Pharmazeutische Zusammensetzung nach Anspruch 17, wobei:

   - der Arzneimittelwirkstoff Lornoxicam oder ein pharmazeutisch unbedenkliches Salz oder Prodrug davon ist; und
   - die alkalische Substanz Histidin, Lysin oder Arginin ist.

21. Pharmazeutische Zusammensetzung nach Anspruch 17, wobei:

   - der Arzneimittelwirkstoff Lornoxicam ist; und
   - die alkalische Substanz Histidin, Lysin oder Arginin ist.

22. Stabile pharmazeutische Zusammensetzungen für die orale Verabreichung, umfassend:

   - ein NSAID oder ein pharmazeutisch unbedenkliches Salz oder Prodrug davon;
   - eine oder mehrere alkalische Substanzen, ausgewählt aus einem Salz, das ein Anion, ausgewählt aus $CO_3^{2-}$, $HPO_4^{2-}$, $PO_4^{3-}$ und ein Kation, ausgewählt aus $Na^+$ und $K^+$, enthält;
   - und ein Bindemittel in Form eines hydrophilen Polymers;

   wobei die pharmazeutische Zusammensetzung mittels eines Verfahrens hergestellt wurde, das ein intensives Mischen in Form eines gemeinsamen Vermahlens und ohne jegliche Verwendung von Flüssigkeit nutzt, wobei das Molverhältnis zwischen dem NSAID oder dem pharmazeutisch unbedenklichen Salz oder Prodrug davon und der/den alkalischen Substanz(en) oder einem Derivat davon im Bereich von 1:100 bis 1:1 liegt.

23. Stabile pharmazeutische Zusammensetzungen für die orale Verabreichung, umfassend:

   - ein NSAID oder ein pharmazeutisch unbedenkliches Salz oder Prodrug davon; und
   - eine oder mehrere Aminosäuren oder ein Derivat davon;

   wobei die pharmazeutische Zusammensetzung mittels eines Verfahrens hergestellt wurde, das ein Mischen des NSAID oder des pharmazeutisch unbedenklichen Salzes oder Prodrugs davon und der einen oder mehreren Aminosäuren oder eines Derivats davon und fakultativ eines oder mehrerer Hilfsstoffe durch gemeinsames Vermahlen ohne die Zugabe einer Flüssigkeit umfasst, wobei das Molverhältnis zwischen dem NSAID oder dem pharmazeutisch unbedenklichen Salz oder Prodrug davon und der/den Aminosäure(n) oder einem Derivat davon im Bereich von 1: 100 bis 1:1 liegt.

24. Pharmazeutische Zusammensetzung nach Anspruch 23, wobei die Aminosäure oder ein Derivat davon Histidin, Lysin oder Arginin ist.

25. Pharmazeutische Zusammensetzung nach Anspruch 22 oder 23, wobei das NSAID Ampiroxicam, Droxicam, Lornoxicam, Meloxicam, Piroxicam, Tenoxicam, Ibuprofen oder Dexibuprofen oder ein pharmazeutisch unbedenkliches Salz oder Prodrug davon ist.

26. Pharmazeutische Zusammensetzung nach Anspruch 22 oder 23, wobei das NSAID Lornoxicam oder ein pharmazeutisch unbedenkliches Salz oder Prodrug davon ist.

27. Pharmazeutische Zusammensetzung nach Anspruch 23, wobei das NSAID Lornoxicam ist und wobei die Aminosäure oder ein Derivat davon Lysin ist.

**28.** Pharmazeutische Zusammensetzung nach Anspruch 23, wobei das NSAID Lornoxicam ist und wobei die Aminosäure oder ein Derivat davon Arginin ist.

**29.** Pharmazeutische Zusammensetzung nach einem der Ansprüche 17-28, wobei die Zusammensetzung ein Opioid oder Triptan in Kombination mit Lornoxicam umfasst.

**30.** Pharmazeutische Zusammensetzung nach einem der Ansprüche 17-29 oder Verfahren nach einem der Ansprüche 1-16, wobei das NSAID oder der Arzneimittelwirkstoff Lornoxicam ist.

**31.** Pharmazeutische Zusammensetzung nach einem der Ansprüche 17-30 oder Verfahren nach einem der Ansprüche 1-16, wobei die Zusammensetzung oder jede orale Darreichungsform 4-12 mg Lornoxicam umfasst.

**32.** Pharmazeutische Zusammensetzung nach einem der Ansprüche 22-31 oder orale Darreichungsform nach einem der Ansprüche 1-16, wobei die Zusammensetzung oder orale Darreichungsform in Form einer komprimierten Tablette vorliegt.

**33.** Pharmazeutische Zusammensetzung nach einem der Ansprüche 17-32 oder Verfahren nach einem der Ansprüche 1-16, wobei das Molverhältnis zwischen Lornoxicam und der/den alkalischen Substanz(en) zwischen 1:100 und 1: 10 liegt.

## Revendications

**1.** Procédé de préparation d'une forme galénique orale comprenant une substance médicamenteuse active à dissolution rapide dans le liquide gastrique, ledit procédé comprenant les étapes suivantes :

a) apport de la substance médicamenteuse active ;
b) apport d'une ou plusieurs substance(s) alcaline(s) ; et
c) mélange de ladite substance médicamenteuse active avec la/lesdite(s) substance(s) alcaline(s) et éventuellement un ou plusieurs excipients par broyage simultané sans ajout de liquide, et éventuellement
d) admixtion d'un ou plusieurs excipients pharmaceutiquement acceptables, et éventuellement
e) compression dudit mélange de c) ou d) pour former un comprimé ;

dans lequel la substance médicamenteuse active présente une solubilité à température ambiante inférieure à 0,1 % p/v dans l'acide chlorhydrique 0,1 N ou présente un pKa inférieur à 5,5 ; et
dans lequel le rapport molaire entre ladite substance médicamenteuse active et la/lesdite(s) substance(s) alcaline (s) se situe entre 1/100 et 1/1.

**2.** Le procédé selon la revendication 1, dans lequel l'étape de broyage simultané (c) est uniquement appliquée à la substance médicamenteuse active et à la/lesdite(s) substance(s) alcaline(s).

**3.** Le procédé selon la revendication 1 ou 2, dans lequel ledit procédé est réalisé dans des conditions sèches excluant l'utilisation de liquide.

**4.** Le procédé selon l'une quelconque des revendications 1 à 3, dans lequel le rapport molaire entre la substance médicamenteuse active et la/les substance(s) alcaline(s) se situe entre 1/100 et 1/10.

**5.** Le procédé selon l'une quelconque des revendications 1 à 4, dans lequel le rapport molaire entre la substance médicamenteuse active et la/les substance(s) alcaline(s) se situe dans la plage de 1/10 à 1/40, et dans lequel ladite substance médicamenteuse est le lornoxicam.

**6.** Le procédé selon l'une quelconque des revendications 1 à 5, dans lequel la/les substance(s) alcaline(s) est/sont soluble(s) dans l'eau avec la caractéristique que 1 part de la substance alcaline peut être dissoute dans un maximum de 100 parts d'eau.

**7.** Le procédé selon l'une quelconque des revendications 1 à 6, dans lequel la/les substance(s) alcaline(s) est/sont un sel d'un acide organique, un sel d'un acide inorganique, une amine organique ou un acide aminé ou un dérivé de ceux-ci.

**8.** Le procédé selon l'une quelconque des revendications 1 à 7, dans lequel la/les substance(s) alcaline(s) est/sont un acide aminé ou un dérivé de celui-ci.

**9.** Le procédé selon la revendication 8, dans lequel l'acide aminé ou un dérivé de celui-ci est la lysine, l'arginine ou l'histidine.

**10.** Le procédé selon la revendication 7, dans lequel l'acide organique et l'acide inorganique présentent un pKa situé dans la plage de 4 à 14.

**11.** Le procédé selon la revendication 7, dans lequel la/les substance(s) alcaline(s) est/sont un sel d'un acide inorganique choisi parmi l'acide carbonique et l'acide phosphorique.

**12.** Le procédé selon l'une quelconque des revendications 1 à 11, dans lequel la substance médicamenteuse active est un AINS ou un sel ou promédicament pharmaceutiquement acceptable de celui-ci.

**13.** Le procédé selon la revendication 12, dans lequel l'AINS est un thiazinecarboxamide ou un sel ou promédicament pharmaceutiquement acceptable de celui-ci.

**14.** Le procédé selon la revendication 12, dans lequel l'AINS est l'ampiroxicam, le droxicam, le lornoxicam, le méloxicam, le piroxicam, l'acide tolfénamique ou le ténoxicam ou un sel ou promédicament pharmaceutiquement acceptable de ceux-ci.

**15.** Le procédé selon l'une quelconque des revendications 1 à 14, dans lequel ledit broyage simultané est assuré par un compacteur à rouleaux.

**16.** Le procédé selon l'une quelconque des revendications 1 à 15, dans lequel le processus de broyage simultané est réalisé à l'aide d'un broyeur à billes.

**17.** Composition pharmaceutique pouvant être obtenue à l'aide du procédé défini dans l'une quelconque des revendications 1 à 16.

**18.** La composition pharmaceutique selon la revendication 17 comprenant :

- un AINS ; et
- un acide aminé ou un dérivé de celui-ci.

**19.** La composition pharmaceutique selon la revendication 17, dans laquelle

- la substance médicamenteuse active est l'ampiroxicam, le droxicam, le lornoxicam, le méloxicam, le piroxicam, le ténoxicam, le bromazépam, l'ibuprofène, l'acide tolfénamique ou le dexibuprofène ou un sel ou promédicament pharmaceutiquement acceptable de ceux-ci ; et
- la substance alcaline est l'histidine, la lysine ou l'arginine.

**20.** La composition pharmaceutique selon la revendication 17, dans laquelle

- la substance médicamenteuse active est le lornoxicam ou un sel ou promédicament pharmaceutiquement acceptable de celui-ci ; et
- la substance alcaline est l'histidine, la lysine ou l'arginine.

**21.** La composition pharmaceutique selon la revendication 17, dans laquelle

- la substance médicamenteuse active est le lornoxicam ; et
- la substance alcaline est l'histidine, la lysine ou l'arginine.

**22.** Composition pharmaceutique stable pour administration orale comprenant :

- un AINS ou un sel ou promédicament pharmaceutiquement acceptable de celui-ci ;
- une ou plusieurs substances alcalines choisies parmi un sel contenant un anion choisi parmi $CO_3^{2-}$, $HPO_4^{2-}$,

$PO_4^{3-}$ et un cation choisi parmi $Na^+$ et $K^+$ ;
- et un liant sous la forme d'un polymère hydrophile ;

dans laquelle ladite composition pharmaceutique a été préparée à l'aide d'un procédé effectuant un mélange intensif sous forme de broyage simultané et sans utiliser aucun liquide, dans laquelle le rapport molaire entre ledit AINS ou sel ou promédicament pharmaceutiquement acceptable de celui-ci et la/lesdite(s) substance(s) alcaline(s) ou un dérivé de celle(s)-ci se situe entre 1/100 et 1/1.

23. Composition pharmaceutique stable pour administration orale comprenant :

- un AINS ou un sel ou promédicament pharmaceutiquement acceptable de celui-ci ; et
- un ou plusieurs acides aminés ou un dérivé de ceux-ci ;

dans laquelle ladite composition pharmaceutique a été préparée à l'aide d'un procédé comprenant le mélange dudit AINS ou sel ou promédicament pharmaceutiquement acceptable de celui-ci avec lesdits un ou plusieurs acides aminés ou un dérivé de ceux-ci et éventuellement un ou plusieurs excipients par broyage simultané sans ajout de liquide, dans laquelle le rapport molaire entre ledit AINS ou sel ou promédicament pharmaceutiquement acceptable de celui-ci et le(s)dit(s) acide(s) aminé(s) ou un dérivé de ceux-ci se situe entre 1/100 et 1/1.

24. La composition pharmaceutique selon la revendication 23, dans laquelle l'acide aminé ou un dérivé de celui-ci est l'histidine, la lysine ou l'arginine.

25. La composition pharmaceutique selon les revendications 22 ou 23, dans laquelle l'AINS est l'ampiroxicam, le droxicam, le lornoxicam, le méloxicam, le piroxicam, le ténoxicam, l'ibuprofène ou le dexibuprofène ou un sel ou promédicament pharmaceutiquement acceptable de ceux-ci.

26. La composition pharmaceutique selon les revendications 22 ou 23, dans laquelle l'AINS est le lornoxicam ou un sel ou promédicament pharmaceutiquement acceptable de celui-ci.

27. La composition pharmaceutique selon la revendication 23, dans laquelle l'AINS est le lornoxicam et dans laquelle l'acide aminé ou un dérivé de celui-ci est la lysine.

28. La composition pharmaceutique selon la revendication 23, dans laquelle l'AINS est le lornoxicam et dans laquelle l'acide aminé ou un dérivé de celui-ci est l'arginine.

29. La composition pharmaceutique selon l'une quelconque des revendications 17 à 28, dans laquelle la composition comprend un opioïde ou un triptan en association avec le lornoxicam.

30. La composition pharmaceutique selon l'une quelconque des revendications 17 à 29 ou le procédé selon l'une quelconque des revendications 1 à 16, dans la/lequel(le) l'AINS ou la substance médicamenteuse active est le lornoxicam.

31. La composition pharmaceutique selon l'une quelconque des revendications 17 à 30 ou le procédé selon l'une quelconque des revendications 1 à 16, dans la/lequel(le) la composition ou chaque forme galénique orale comprend 4 à 12 mg de lornoxicam.

32. La composition pharmaceutique selon l'une quelconque des revendications 22 à 31 ou la forme galénique orale selon l'une quelconque des revendications 1 à 16, dans laquelle ladite composition ou forme galénique orale se présente sous la forme d'un comprimé.

33. La composition pharmaceutique selon l'une quelconque des revendications 17 à 32 ou le procédé selon l'une quelconque des revendications 1 à 16, dans la/lequel(le) le rapport molaire entre le lornoxicam et la/les substance(s) alcaline(s) se situe entre 1/100 et 1/10.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9641646 A
- WO 9532737 A
- WO 9909988 A
- EP 1109534 B1
- WO 9912524 A
- EP 1109534 A
- WO 15195 A
- JP 3240729 B
- EP 792147 A